# EUROPEAN PATENT APPLICATION

(11) **EP 4 685 482 A1**
(43) Date of publication of application: **28.01.2026**
(21) Application number: 24773743.0
(22) Date of filing: 20.03.2024
(51) Int. Cl.: G01N 33/48

(54) **METHOD FOR SELECTING DRUGS FOR THE TREATMENT OF MENTAL HEALTH PATHOLOGIES**

(30) Priority: 22.03.2023 AR P230100703
(71) Applicant: Neomente Corp, Chevy Chase, Maryland 5417 (US)
(72) Inventor: YAHIA, Ariel Angel, Ciudad Autónoma de Buenos Aires (AR); NIELSEN, María Gabriela, Ciudad Autónoma de Buenos Aires (AR)
(74) Representative: Hoffmann Eitle
(86) International application number: PCT/CL2024/050019
(87) International publication number: WO 2024/192543

(57) **Abstract**

The present invention relates to a method for selecting drugs for the treatment of mental health pathologies that, given its general features, can be applied to a broad spectrum of disorders, such as Major Depressive Disorder (MDD), Bipolar Disorder (BD), Attention Deficit Disorder (ADD), Psychosis (PS), Generalised Anxiety Disorder (GAD) and Social Anxiety Disorder (SAD), by applying an algorithm that combines the patient's genetic information (genotype), clinical and blood parameters (phenotype), and characteristics of the candidate drugs to optimise the pharmacological treatment.

## Description

### TECHNICAL FIELD OF THE INVENTION

This invention relates to a method for the selection of drugs for the treatment of mental health pathologies which, due to its general characteristics, can be applied to a wide range of disorders, such as Major Depression Disorder (MDD), Bipolar Disorder (BPD), Attention Deficit Disorder (ADD), Psychosis (PS), Generalized Anxiety Disorder (GAD), and Social Anxiety Disorder (SAD) by applying an algorithm that combines patient genetic information (genotype), blood and clinical parameters (phenotype), and characteristics of candidate drugs to optimize pharmacological treatment.

### STATE OF THE ART AND PROBLEMS TO SOLVE

### (Background of the invention)

The prevalence of mental illnesses is significant, impacting approximately one in every two individuals at some point in their lives. The economic ramifications of poor mental health are substantial, estimated at over 4.2% of the GDP of OECD countries, encompassing both direct treatment costs and indirect costs linked to decreased employment rates and productivity. Mortality rates for individuals with schizophrenia and bipolar disorder are notably higher in most nations compared to the general population. Additionally, the consumption of antidepressant medications has surged by nearly 50% in OECD countries between 2011 and 2021, with a further 10% increase observed between 2019 and 2021. (Organization for Economic Co-operation and Development [OECD], 2023).

Depression and anxiety, two prevalent mental health conditions, collectively incur an annual global economic burden of USD 1 billon each (The Lancet Global Health, 2020). A significant proportion (75%) of mental health disorders affecting approximately 970 million individuals worldwide are attributable to schizophrenia, depressive disorders, bipolar disorder, anxiety disorders, and attentiondeficit/hyperactivity disorder (World Health Organization [WHO], 2022).

Traditionally, the field of psychiatry has relied on a trial-and-error approach to medication prescription, resulting in elevated treatment costs and failures. Incorporating information about a patient's genetic background can aid clinicians in identifying appropriate treatment options by predicting potential pharmacotherapeutic responses or adverse effects (Bishop et al., 2023; Radosavljevic et al., 2023; Van Westrhenen & Ingelman-Sundberg, 2021). Therefore, there is a pressing need to implement strategies to predict or mitigate these inadequate responses. In this context, leveraging biomarkers acknowledged in the scientific literature for their influence on pharmacological response, this study seeks to integrate them to furnish physicians with a tool facilitating the selection of the most suitable drug for each patient.

Currently, there is a variety of drugs available for the treatment of mental health pathologies. Among them, we can highlight for their therapeutic achievements the use of antidepressants like serotonin reuptake inhibitors (SSRIs), norepinephrine reuptake inhibitors (NRIs), combined serotonin and norepinephrine reuptake inhibitors (SNRIs), monoamine oxidase inhibitors (MAOls), mood stabilizers, stimulants, antipsychotics, non-stimulants, benzodiazepines, non-benzodiazepines anxiolytics, anesthetics, or other psychoactive compounds. There are also drugs not yet widely established as standard treatments for psychiatric disorders, and their use in this field will require further research and clinical evaluation. Despite these varied therapeutic options, many patients do not respond, have a delayed response, or only partially respond to treatment, so patients must try various treatment options for weeks or months before receiving the benefits of appropriate treatment.

Furthermore, for example, individuals with major depression who are initially prescribed pharmacological antidepressant therapy do not always experience timely remission of depression symptoms over time. Most clinical trials report remission rates of 22% to 40%, while efficacy studies including representative samples of depressed patients closer to clinical practice report lower remission rates, around 11% to 30% (Mendlewicz, 2008).

Where an illness does not respond despite an adequate course of treatment, it is generally termed treatment-resistant. Treatment resistance is now recognized across a range of psychiatric disorders, including schizophrenia, MDD, and bipolar affective disorder. Treatment resistance affects 20-60% of patients with psychiatric disorders and is associated with increased healthcare burden and costs up to ten-fold higher relative to patients in general (Howes et al., 2021).

Everyone responds to drugs differently. The way a person responds to a drug is affected by many factors, including genetic makeup, age, body size, use of other drugs and dietary supplements, (such as medicinal herbs), consumption of food (including beverages), presence of diseases (such as kidney or liver disease), development of tolerance and resistance (Lynch, 2022).

The relevant pharmacokinetic genes for psychiatry include those encoding for cytochrome P450 enzymes and glucuronidases involved in phase 2 drug metabolism. Variants in pharmacodynamic genes affect how a drug impacts the body, for example, the degree of activation of a particular receptor at a specific concentration of a drug. Approximately 40% of the variance in response to antidepressants may stem from common genetic variants, with effects identified more consistently derived from variation in pharmacokinetic genes (Tansey et al., 2013).

Prior inventions have suggested the analysis of specific single nucleotide polymorphisms (SNPs) for diagnosing depression (McMahon et al., 2008), described a method for optimizing and individualizing the selection and dosage of medications (Glauser et al., 2006), or provided computer-assisted methods for selecting an antidepressant medication for a patient (Mrazek et al., 2019). These methods utilize a combined genotype comprising at least three genes to guide medication selection.

However, there is significant interindividual variability in the response to most neuropsychiatric medications. The choice and dosage adjustment of medication are often experimental rather than individualized. The reason why physicians do not consider the genetic profile and non-heritable factors as causes of variability and inter-variability to plan treatment is the absence of applicable algorithms that interpret patient characteristics into pharmaco-clinical recommendations. Therefore, there is a need for a technique that allows for dose individualization to optimize the efficacy and safety of psychotropic medication in an informative, economical, and effective manner.

### BRIEF DESCRIPTION OF THE INVENTION

The present invention describes a METHOD FOR SELECTING DRUGS FOR THE TREATMENT OF MENTAL HEALTH PATHOLOGIES comprising the following steps:
a- Three groups of biomarkers are obtained from a patient through i) a group of Biomarkers 1, ii) a group of Biomarkers 2, and iii) a group of Biomarkers 3; where
   Group of Biomarkers 1 corresponds to the patient's clinical parameters relevant to the treatment of each pathology and its differential diagnosis;
   Group of Biomarkers 2 corresponds to the patient's blood parameters relevant to pharmacological response; and
   Group of Biomarkers 3 corresponds to the patient's genetic conditions relevant to pharmacological treatment;
b- taking into account the values of Groups of Biomarkers 1, 2, and 3; attributes are determined for a Multicriteria Decision Analysis;
c- considering the results of the previous step, the weight of each attribute is determined for the Multicriteria Decision Analysis;
d- considering the previous results, scores are assigned to each attribute for each drug of interest;
e- a final calculation of the score of each drug is carried out by multiplying the scores of each attribute by its weight and summing the obtained values; and
f- a list of drugs is provided, of which those with higher scores have a higher probability of response and a lower risk of adverse effects for the patient.

### BRIEF DESCRIPTION OF THE FIGURES

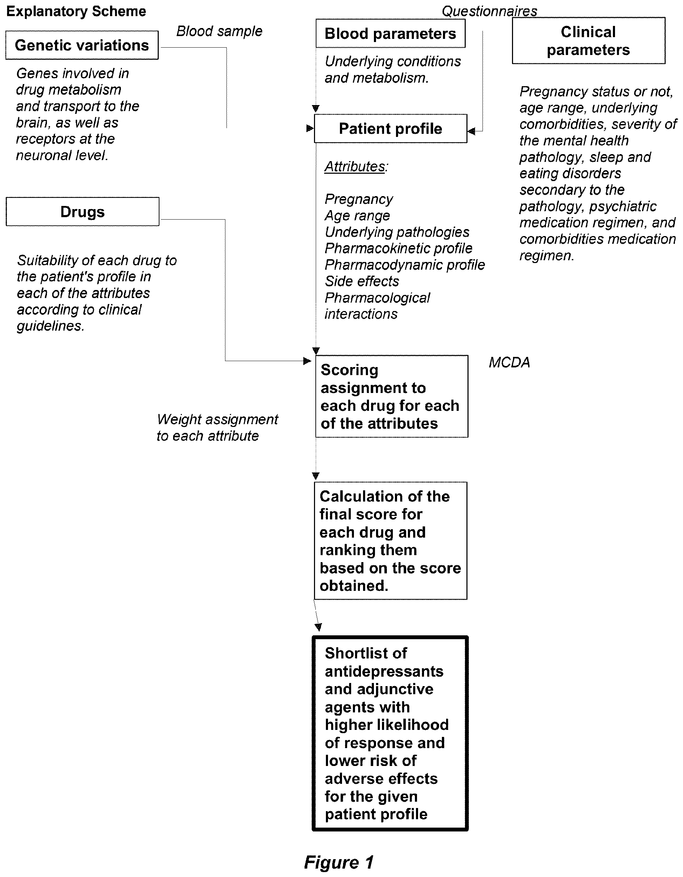

Figure 1 shows an explanatory diagram of the method described in the present invention.
Figure 2 shows the 17-item Hamilton Depression Rating Scale (SIGH-D-17) in two groups; group I: Usual treatment; group II: Guided by the Clinical Decision Support System. Follow-up was carried out using the SIGH-D-17 scale in both groups at the beginning of treatment, at 8 weeks, and at 12 weeks.

### DETAILED DESCRIPTION OF THE INVENTION

The invention relates to a method for selecting drugs for the treatment of mental health pathologies, which utilizes a specific algorithm applied to patient data provided or received, as well as drug data. Part of this data is reported by the patient (self-reported) and reported by the treating medical professional in the method requisition form.

The method is characterized by analyzing three groups of biomarkers, with the data obtained being incorporated into a processor. It also contains data on compounds, drugs, and active ingredients, and comprises efficacy and safety data, pharmacokinetic and pharmacodynamic characteristics, and pharmacological interactions. The data is organized in tables or lists that, when received by the processor, execute instructions that translate into functional information that optimizes the selection of one or more drugs for safe and effective treatment, minimizing adverse events, and in some cases death, depending on the patient's characteristics.

Like the prior art, this invention considers SNPs and variants of longer nucleotides. However, unlike all the aforementioned prior art, it also interrelates genomic, clinical, and blood data.

Using multi-attribute decision-making techniques and evidence-based clinical and research guidelines, a score is assigned to each medication obtained as a linear combination of weighted attributes. Once the scoring assignment process is completed, the medications are ranked based on these scores in a final shortlist. The system also provides the medical professional with legends related to each drug-attribute pair.

The algorithm can be summarized in the following steps:
1. Identification and evaluation of:
   1.1. Clinical parameters of a patient relevant to the treatment of the pathology and its differential diagnosis (Biomarker Group 1).
   1.2. Blood parameters of a patient relevant to pharmacological response (Biomarker Group 2).
   1.3. Genetic variations of a patient relevant to pharmacological treatment (Biomarker Group 3).
   1.4. Relevant medications for the treatment of the pathology according to applicable clinical guidelines and,
2. Application of a multi-attribute decision-making technique to rank medications according to their predicted performance in treating the patient's neuropsychiatric pathology.

The information contained in the three biomarker groups and the pharmacological profiles recommended by clinical guidelines for evaluating neuropsychiatric pathology are combined using multiple-criteria decision-making techniques to create a shortlist of medications.

Through the analysis of multiple attribute selection, the system ranks medications according to the likelihood of being recommended in the neuropsychiatric pharmacological treatment of the patient. A Multi-Criteria Decision Analysis (MCDA) technique A Simple Multi-Attribute Rating Technique (SMART) is used for this purpose. It involves a systematic approach to decision-making that considers various factors or attributes simultaneously to evaluate and compare different options (Department for Communities and Local Government [DCLG], 2009; Edwards & Barron, 1994; Hansen & Devlin, 2019).

Attributes such as the patient's pharmacokinetic and pharmacodynamic profiles, blood parameters, age range, pregnancy status, underlying pathologies, and drug interactions are weighted and combined. The drug-drug interaction (DDI) attribute is an optional attribute that assesses interactions among all drugs comprising the patient's current pharmacological regimen. It assigns a score to each interaction based on the Risk or Severity of the Interaction. While the score of this attribute is reported to the medical professional, it is not included in the simple multi-attribute rating technique calculation.

The hierarchized shortlist of first-, second-, and third-line medication, including adjunctive agents, according to international clinical guidelines includes antidepressants, mood stabilizers, antipsychotics, anxiolytics, hypnotics, or stimulants according to the pathology being addressed. For example, in the case of major depression, the hierarchized shortlist of drugs, such as antidepressants, is also accompanied by a hierarchized shortlist of adjunctive antipsychotics and psychostimulants.

The method for selecting neuropsychiatric drugs for the treatment of mental health pathologies of the present invention is defined as follows:
1. Three biomarker groups of a patient are obtained where:
   1.1. Biomarker Group 1 corresponds to the clinical parameters of the patient relevant to the treatment of the pathology and its differential diagnosis.
   1.2. Biomarker Group 2 corresponds to the blood parameters of the patient relevant to pharmacological response and,
   1.3. Biomarker Group 3 corresponds to the genetic variations of the patient relevant to pharmacological treatment.
2. Considering the values of Biomarker Groups 1, 2, and 3, attributes are determined for the Multi-Criteria Decision Analysis.
3. Considering the results from the previous step, the weights of the attributes are determined for the Multi-Criteria Decision Analysis.,
4. Considering the previous results, scores are assigned to each attribute for each drug of interest.
5. The final score for each drug is calculated by multiplying the scores of each attribute by its weight and summing the obtained values.
6. A list of drugs is provided, among which those with higher scores have a higher probability of response and a lower risk of adverse effects for the patient.

### METHOD DESCRIPTION

### Biomarker Group 1

### Phenotypes - Clinical parameters

It includes clinical parameters and phenotypes obtained from the patient through self-reporting or reported by the attending clinician responsible for the patient's treatment. Once the information is gathered, the following parameters are defined, which are the clinical parameters considered by the drug selection algorithm: Patient ID, Gender, Age, Pregnancy, Age range, Underlying pathology, Bipolar spectrum, Body Mass Index (BMI), Sleep disorder, Suicidal ideation, Pathology severity, Weight change, Smoking, Cognitive effects, Previous medications, Current medications, Adverse events induced by past or current medication, Information on whether patient is experiencing subtherapeutic response with current medication.

The age range is defined as follows: Pediatric: Age under 18 years, Adult: Age between 18 and 65 years, and Geriatric: Age over 65 years.

Underlying pathologies,, for example may include the following: Epilepsy, Cardiac, Urinary dysfunction/Prostate hypertrophy, Immunological, Polycystic ovary syndrome, Neurological (multiple sclerosis, stroke, Organic brain syndrome), Parkinson's, Chronic anemia, Diabetes, Hypothyroidism, Hyperthyroidism, Biliary cirrhosis, Pheochromocytoma, Neuroblastoma, Closedangle glaucoma, Current or past diagnosis of bulimia or anorexia nervosa, Central nervous system (CNS) tumor, Known prolongation of QT interval or congenital long QT syndrome, Hypertension, Renal failure, Acute alcohol or benzodiazepine withdrawal, Liver failure, Lactose intolerance, Problematic alcohol consumption, Hepatic cirrhosis, Risk of bleeding, Elevated intraocular pressure, Hypotension, Dementia, Porphyria and Adrenal insufficiency, untreated pituitary insufficiency, and thyrotoxicosis, Myasthenia gravis, Severe respiratory failure, Sleep apnea syndrome.

Values compatible with Bipolar spectrum is a parameter that is defined if the patient presents the following two conditions:
1. Family history of bipolar disorder, i.e., when the answer to the question of the Patient Questionnaire is affirmative: Has any of your siblings, parents, uncles, and/or grandparents been diagnosed with bipolar disorder? or Family history of suicide, i.e., when the answer to the additional question of the Patient Health Questionnaire is affirmative: Has any of your siblings, parents, uncles, and/or grandparents died by suicide? or Have you had a hypomanic episode after taking an antidepressant?
2. It exceeds the cutoff score on the Hypomania Symptom Checklist (HCL32) (Angst et al., 2005) or exceeds the cutoff score on the Mood Disorder Questionnaire (MDQ) (Hirschfeld et al, 2000).

BMI is constructed by combining two anthropometric variables: weight and height.

The clinician reports the Diagnosis, based on which, the psychometric scale (s) to be used to measure the Severity of the Pathology is determined, for example:
1. MDD and BPD in a current depressive episode or depressive episode with mixed features: Hamilton Depression Rating Scale-17 items (SIGH-D-17) (Hamilton, 1960; Kobak, 2004), Patient Health Questionnaire-9 (PHQ-9) (Kroenke et al., 2001), HCL-32 and MDQ.
2. BPD in a current Hypomanic or manic episode and Hypomanic or manic episode with mixed features: Young Mania Rating Scale (YMRS) (Young et al.,1978), HCL-32, and MDQ.
3. GAD and SAD: Hamilton Anxiety Rating Scale (HAM-A) (Hamilton, 1959; Maier et al., 1988) and Anxiety Sensitivity Index-3 (ASI-3) (Taylor et al., 2007).
4. PS: Positive and Negative Syndrome Scale (PANSS) (Kay, 1960; Kay et al. 1987; Kay et al., 1988).
5. ADD: ADHD Rating Scale 5 (ADHD-RS-5) (Faries et al., 2001; Nasser et al., 2021).

In the case of BPD, also the current episode diagnosis is required (American Psychiatric Association [APA], 2013). In the case of PS, first-episode or multi-episode needs to be reported for treatment choice (Hasan et al., 2017). Sleep disorder is a parameter defined as insomnia if the answer to question 1.3 of PHQ9 is affirmative: "Have you had trouble falling or staying asleep, or have you been sleeping too much?" and the patient selects any of the following alternatives: "I sleep a little less than usual," "I sleep much less than usual," or "I wake up 1-2 hours earlier and cannot fall back asleep." Conversely, it is defined as hypersomnia if the answer to question 1.3 of the PHQ9 is affirmative and the patient selects any of the following alternatives: "I sleep a little more than usual," "I sleep much more than usual," or "I sleep most of the day."

Weight change is defined based on self-reported information. It can be Weight loss (decrease of more than 5% of weight in 1 month) or Weight gain (increase of more than 5% of weight in 1 month).

Smoking (Tobacco use) is defined when the patient responds affirmatively to any of the following three questions: "Do you smoke less than 10 cigarettes per day?", "Do you smoke more than 10 cigarettes per day?" or "Do you live in a closed environment with a person who smokes more than ten cigarettes per day?"

Previous and current medications are obtained from clinical parameters reported by the attending physician responsible for the patient's treatment, and the information includes Names of medications, Any adverse reactions experienced by the patient, and Any subtherapeutic responses to dosage increases/lack of response. The patient is also asked to report current medications.

### Biomarker Group 2

### Phenotypes - Blood parameters

These data are reported by the clinical analysis laboratory and can include: White Blood Cell Count (Leukocytes), Hemoglobin, Hematocrit, Neutrophils, Eosinophils, Basophils, Lymphocytes, Monocytes, Glucose, Urea, Creatinine, Total Cholesterol, Aspartate Aminotransferase (AST), Alanine Aminotransferase (ALT), Total Bilirubin, Alkaline Phosphatase, Total Proteins, Albumin, Thyroid Stimulating Hormone (TSH), Triiodothyronine (T3), Free Thyroxine (T4L), Triglycerides, HDL Cholesterol, LDL Cholesterol, Platelets, Prothrombin Concentration, International Normalized Ratio (INR), Activated Partial Thromboplastin Time (APTT), Sodium, Potassium.

Once the information is collected, the following parameters are defined as the blood parameters considered by the drug selection algorithm:
They are elevated or decreased Hematocrit if the value is higher or lower than the corresponding normal parameter based on whether the patient is male, female, pregnant female, or pediatric age range.

Decreased Hemoglobin if the value is lower than the corresponding normal parameter based on whether the patient is a male, female, or pregnant female.

Values compatible with Anemia are defined by abnormal levels of red blood cells (lower than normal), hemoglobin levels (lower than normal), hematocrit levels (lower than normal), and mean corpuscular volume levels (higher or lower than normal).

Values compatible with Leukopenia are defined if the white blood cell count is lower than the corresponding normal parameter.

Values compatible with Neutropenia are defined if the neutrophil count is lower than the corresponding normal parameter.

Values compatible with Liver Dysfunction are defined when any of the transaminase levels (Bilirubin, AST, ALT, Alkaline Phosphatase, Proteins, and Albumin) exceed the upper limit (maximum value) of the normal range but do not exceed 3 times the maximum value.

Values compatible with Liver Failure are defined when any of the transaminase levels exceed three times the maximum value.

Values compatible with Kidney Dysfunction are defined when the Urea or Creatinine level exceeds the normal value.

Values compatible with Hypothyroidism are defined when the level of TSH is elevated and T4L is decreased.

Values compatible with Hyperthyroidism are defined when the level of TSH is decreased and T4L is elevated.

Values consistent with Renal Insufficiency are defined when the glomerular filtration rate is below 30 according to the Chronic Kidney Disease Epidemiology Collaboration (CKD-EPI) equation (Levey et al., 2009).

Values compatible with Risk of Bleeding are defined when any of the following criteria are met:
1. Platelet count lower than normal (thrombocytopenia), or
2. High INR values, high Partial Thromboplastin Time values, or Prothrombin Concentration lower than normal parameters.

Values compatible with the Risk of Venous Thromboembolism are defined when any of the following criteria are met:
1. Platelet count higher than normal (thrombocytosis), or
2. Low INR values, low Partial Thromboplastin Time values, or Prothrombin Concentration higher than normal parameters.

Values compatible with Hyponatremia are defined when sodium values are lower than the normal value.

Values compatible with Hypokalemia are defined when potassium values are lower than the normal value.

### Biomarker Group 3

### Genotypes

The patient's genotype data is obtained through the results of a genetic analysis.

Most medications used in psychiatric treatment undergo hepatic metabolism, with exceptions like lithium for example, which is solely eliminated through the kidneys. Various genes encoding oxidative (Phase 1) and conjugative (Phase 2) metabolizing enzymes carry variants known to impact their activity. Additionally, genetic differences in drug transporters expressed in the liver, gut, and at the blood-brain barrier can modify drug distribution, thus influencing their pharmacokinetic profile (Bousman et al., 2020).

Many genetic variants in drug-metabolizing enzymes and transporters are pertinent to psychiatry, with associations significant enough to be noted on drug labels and incorporated into prescribing guidelines by the Clinical Pharmacogenetics Implementation Consortium (CPIC) (CPIC, 2024) and the Dutch Pharmacogenetics Working Group (DPWG, 2023). The International Society of Psychiatric Genetics recommends HLA-A and HLA-B testing before using carbamazepine and oxcarbazepine and suggests that genetic data for CYP2C19 and CYP2D6 would be particularly valuable for individuals who previously had inadequate response or adverse reactions to antidepressants or antipsychotics (Carvalho et al., 2020; International Society of Psychiatric Genetics [ISPG], 2019).

The CYP superfamily is widely considered the primary enzyme system for drug metabolism. Allelic variants of CYP genes are commonly denoted using the star (*) nomenclature. Genotypes, reported as star diplotypes (e.g., CYP2D6*1/*2), are then translated into metabolizer phenotypes. The most used classification system categorizes individuals into ultrarapid metabolizers (UMs), rapid metabolizers (RMs), normal metabolizers (NMs), intermediate metabolizers (IMs), and poor metabolizers (PMs), based on their genetic makeup, which can impact how they respond to medications. In this context, "activity" refers to the metabolic capacity of an enzyme, encompassing catalytic activity and enzyme abundance (Bousman et al., 2020; Caudle et al., 2017).

Findings have shown that genetic variants like for example CYP2C19 and CYP2D6 are associated with antidepressant blood concentrations, adverse drug reactions, and, to a lesser extent, clinical outcomes such as treatment discontinuation or symptom response (Braten et al., 2019; Jukić et al., 2018). Seventeen antidepressants have been included in published pharmacogenomics-based prescribing guidelines or product labels for associations with CYP2C19 and/or CYP2D6. The CPIC guidelines for CYP2C19 PMs suggest a 50% reduction of the recommended starting dose of citalopram, escitalopram, sertraline, and tertiary amine tricyclic antidepressants (e.g., amitriptyline); whereas RMs/UMs treated with citalopram, escitalopram, and tertiary amine tricyclic antidepressants would likely have inadequate treatment response due to inadequate circulating antidepressant blood levels and thus may benefit from an alternative antidepressant. For CYP2D6 PMs, CPIC recommends up to a 50% reduction of most tricyclic antidepressants, fluvoxamine, and paroxetine doses, while for UMs, it is advised to select an alternative antidepressant that is not predominantly metabolized by CYP2D6 (Bousman et al., 2020; Hicks et al., 2015; Hicks et al., 2017). In addition, the DPWG recommends reduced dosing (amount unspecified) of venlafaxine for CYP2D6 PMs and up to 150% increased dosing for UMs (DPWG, 2023; Swen et al., 2011).

Like the majority of psychiatric drugs, most antipsychotics are hepatically metabolized by one or more CYP450 enzymes. Ten antipsychotics have product labels or prescribing guidelines that offer selection or dosing recommendations based on CYP2D6 metabolizer status. For all of these drugs, the guidelines or product labels recommend that CYP2D6 PMs receive lower starting doses or an alternative drug not primarily metabolized by CYP2D6.³⁸ In addition, the DPWG guidelines recommend reductions in the starting dose for pimozide and zuclopenthixol among CYP2D6 IMs, while for UMs they recommend the use of an alternative drug or titration to the maximum dose for haloperidol, risperidone, and zuclopenthixol. The Food and Drug Administration (FDA) product label for clozapine suggests CYP2D6 PMs may require a dose reduction (Bousman et al., 2020).

Product labels and prescribing guidelines are available for antiepileptics used as mood stabilizers like carbamazepine and oxcarbazepine. CPIC recommends use of alternative medications for individuals who test positive for HLA-A*31:01 (carbamazepine) or HLA-B*1502 (carbamazepine, oxcarbazepine). FDA, Health Canada's Special Access Program (HCSC), and the Pharmaceuticals and Medical Devices Agency (PMDA) product labels include language that valproic acid is contraindicated or recommend genetic tests before prescribing valproic acid to individuals suspected (e.g., by family history) of having certain rare metabolic disorders. Sequencing of the gene POLG (mitochondrial DNA polymerase γ) is recommended in patients suspected of having a mitochondrial disorder, while patients suspected of having a urea cycle disorder should be screened for mutations in the genes OTC (ornithine transcarbamylase) and CPS1 (carbamoyl-phosphate synthase 1). The use of valproic acid by these individuals can induce liver toxicity, hyperammonemia, and encephalopathy (Bousman et al., 2020; Finsterer & Segall, 2009).

Most anxiolytic/hypnotic medications are preferentially metabolized by CYP3A4, CYP3A5, and CYP2C19 (Whirl-Carrillo et al., 2012; Whirl-Carrillo et al., 2021). Stimulants, including methylphenidate and the non-stimulant atomoxetine, are generally the first-line treatments to alleviate core ADHD symptoms. CYP2D6 is noted as a pharmacogenomic biomarker that may help guide treatment with atomoxetine. Official FDA product labeling, CPIC, and DPWG all note the clinical relevance of CYP2D6 genetic variation for atomoxetine prescribing (Brown et al., 2019; Swen et al., 2011). In the product labeling, patients taking a CYP2D6 inhibitor or who are known CYP2D6 PMs are recommended to start at the same dose as NMs but to approach dose escalation differently by only considering increases after 4 weeks if the drug is tolerated and symptoms do not improve.

To carry out this invention, a panel of genes is defined which may contain the following pharmacokinetic variants:
CYP1A2: rs762551 (-163C>A), variant defining the CYP1A2*1F haplotype associated with altered phenotype. It is considered to have increased activity (UM) due to enhanced expression induction. The variation rs2069514 (-3860G>A) defines the CYP1A2*1C haplotype, which has decreased activity. The variant rs12720461 (-729 C>T) along with rs762551 and rs2069526 (-739 T>G) define the CYP1A2*1K haplotype, which has decreased activity. Also, rarer variants that define the haplotypes of decreased in vivo activity CYP1A*23, CYP1A2*4, and CYP1A*27 or decreased in vitro activity CYP1A*28, CYP1A2*11, CYP1A2*15, and CYP1A2*16 may be included (Pharmacogene Variation Consortium [PharmVar], n.d.; Thorn et al., 2012).
CYP2B6: rs2279343 (18053A>G) variant defines the CYP2B6*4 haplotype associated with increased activity. The genetic variation rs3745274 (516 G>T) is associated with decreased function of the CYP2B6*9 haplotype. The rs3745274, along with rs2279343, are associated with decreased function of the CYP2B6*6 haplotype. The variation rs28399499 (21011T>C) defines the CYP2B6*18 associated with no enzymatic activity. The rs3211371 (1459C>T), along with rs2279343 and rs3745274, are associated with decreased function of the CYP2B6*7 allele. The variant rs34223104 (-827T>C) defines the CYP2B6*22 haplotype of increased function. The rs3745274 (516 G>T), along with rs2279343 and rs3211371, are associated with decreased function of the CYP2B6*7 haplotype. Also, rarer variants associated with no function haplotypes CYP2B6*8, *12, *13, *24, *28, *30, *35, *37, and *A38 or associated with decreased function haplotypes *19, *20, *26, *29, *34 and *36, may be included (PharmVar, n.d.; Thorn et al., 2010).
CYP3A4: rs35599367 (15389 C>T) defines the CYP3A*22 haplotype associated with decreased activity. A rarer variant rs67666821 (25898_25899insA) that defines the no-function haplotype CYP3A*20 may be included (PharmVar, n.d.).
CYP3A5: rs776746 (6981A>G), rs10264272 (14685G>A), and rs41303343 (27126_27127insT) define the no function haplotypes CYP3A5*3, CYP3A5*6, and CYP3A5*7, respectively, associated with decreased activity. A rarer variant rs67666821 (25898_25899insA) that defines the no-function haplotype CYP3A*20 may be included (PharmVar, n.d.; Pratt et al., 2023).
CYP2D6: There are several genetic variants associated with enzyme activity deficiency, including haplotypes *3, *4, *5, and *6; rs35742686 (2550delA), rs3892097 (1847G>A), CYP2D6 full gene deletion, and rs5030655 (1708delT), respectively. Genetic variations determining decreased enzyme activity are rs1065852 (100C>T) and rs1135840(4181G>C), defining haplotype *10, rs28371706 (1022C>T), rs1135840(4181G>T), and rs16947 (2851C>T), defining haplotype *17, rs55030656 (2616delAAG) defining haplotype *9, and CYP2D6*41 defined by rs28371725 (2989G>A), rs1135840 (4181G>T), and rs16947 (2851C>T). The enzyme exhibits ultrarapid activity in response to duplications or multiplications of haplotypes *1 and *2. Other rarer genotypes, reported as star haplotypes (e.g., CYP2D6 *29) with an allele clinical function can be included in the panel (PharmVar, n.d.; Nofziger et al., 2019; Owen et al., 2009; Whirl-Carrillo et al., 2012; Whirl-Carrillo et al., 2021).
CYP2C19: There are several genetic variants associated with enzyme activity deficiency, including rs4244285 (19154G>A) to define haplotype *2; rs3758581 (80161A>G) and rs4986893 (179486G>A) for haplotype *3; and rs3758581 (80161A>G) and rs28399504 (1A>G) to define haplotype *4. Genotype *17 defines rapid enzyme metabolism through the defining polymorphism rs12248560 (806C>T). Other rarer genotypes, reported as star haplotypes, that are translated into clinically relevant CYP2C19 metabolizer phenotypes can be included (PharmVar, n.d.; Scott et al., 2012; Whirl-Carrillo et al., 2012; Whirl-Carrillo et al., 2021).
CYP2C9: The genetic variant associated with enzyme activity deficiency is rs1057910 (42614A>C) and defines haplotype CYP2C9*3; rs1799853 (3608C>T) defines haplotype CYP2C9*2 and decreased enzyme activity. Other rarer genotypes, reported as star haplotypes, that are translated into clinically relevant CYP2C9 metabolizer phenotypes can be included (PharmVar, n.d.; Van Booven, 2010).

Genes that encode conjugative enzymes are annotated at PharmGKB Level 3 clinically, outlining variant-drug combinations with limited evidence supporting their association. UDP-glucuronosyltransferase (UGT) also exhibits Level 3 evidence for medications like lamotrigine, valproic acid, and oxcarbazepine (Gong et al., 2021; Whirl-Carrillo et al., 2012; Whirl-Carrillo et al., 2021). Similarly, catechol-O-methyltransferase (COMT) shows Level 3 evidence for drugs such as venlafaxine and serotonin reuptake inhibitors (SSRIs). The P-glycoprotein (ABCB1) drug transporter also presents Level 3 evidence for medications like haloperidol, and methylphenidate, as well as various mood stabilizers and antidepressants. Although robust clinical evidence of drug-gene interaction is still lacking, these genes are significant when one drug in an individual's regimen influences their ability to metabolize another drug. Phenoconversion refers to the discrepancy between an individual's genotype-based prediction of drug metabolism and their actual capacity to metabolize drugs due to non-genetic factors, such as co-medication. The panel can include UGTs like UGT1A1, UGT1A4, UGT2B15, COMT, and ABCB1 (Manning & Blumenthal, 2023).

Pharmacodynamics pertains to the biochemical, cellular, and physiological effects of medications and their mechanism of action. In psychiatric pharmacogenomics, the historical focus has been on variations in genes encoding neurotransmitter receptors and reuptake transporters situated on preor postsynaptic cell membranes. Recently, attention has expanded to encompass genes involved in signal transduction, gene transcription, and protein folding and trafficking (Bousman et al., 2020). The gene panel may include the following pharmacodynamic variants: ABCB5, ABCC2, ABCG2, ACE, ADCY1, ADGRL3, ADM, ADRA2A, AHR, ALDH1L1, ANKK1, ANO2, ARHGAP12, ARHGEF28, ARSA, ASIC2, ATP10A, BDNF, BMP5, CACNA1A, CACNA1C, CACNG2, CDH23, CES1, CHURC1, CMTM8, CNR1, COL1A1, COL26A1, COMT, CORO7, CREB1, CRH, CRHR1, CRHR2, CRY1, CTNNA3, DBH, DRD1, DRD2, DRD3, DTNBP1, ELP5, EPHX1, EPHX2, ESYT2, ETFDH, FAAH, FAM177A1, FAM178B, FARP2, FCN2, FHIT, FKBP5, FXR2, GABRA1, GABRA6, GABRP, GABRQ, GAL, GDNF, GGH, GLDC, GNB3, GNB3, P3H3, GRAMD1B, GRIA3, GRIK3, GRIN2B, GSK3B, HLA-A, HLA-B, DRB1, HSPA1L, HTR1A, HTR1B, HTR2A, HTR7 RPP30, HTT, INVS, KMT2E, LEPR, LINC01592, MC1R, MDGA2, MIEF2, MTHFR, MTRF1L, MYO1H, NBEA, NCAM1, NFIB, NR1D1, THRA, NR1I2, NRXN1, NTRK2, OPCML, OPRM1, OR52E2, OR52J2P, OR52J3, PAPLN, PARP11, PDIA2, PEBP4, PIGM, POLG, PON2, PURA, PYROXD2, RABEP1, RAPGEF5, REEP5, RGS17, RORA, RRP7A, SACM1L, SCN1A, SCN2A, SENP3, SERPINE1, SH2B1, SLC18A2, SLC22A1, SLC6A2, SLC6A4, SOD2, SPSB2, SRP19, STRBP, TEX10, TH, TPH1, TPH2, TREML4, TRIB3, TTC37, UST, ZDHHC7, ZNF134, ZNF211, ZNF385D, ZNF565, ZNF804A, a list that will be updated as scientific evidence progresses in specialized literature.

### Drugs

According to the clinical guidelines for the treatment of mental health pathologies, the following drugs are some of those that can be used for treatment and therefore considered in this algorithm and any Clinical Decision Support System (CDSS) based on it:
**Anxiolytics** can include Alprazolam, Bromazepam, Buspirone, Diazepam, Hydroxyzine and others;
**Mood Stabilizers** can include Lithium and others;
**Antiepileptics** used as Mood Stabilizers can include Carbamazepine, Lamotrigine, Oxcarbazepine, Valproic Acid, and others;
**Antidepressants** like Amitriptyline, Amisulpride, Citalopram, Clomipramine, Desipramine, Desvenlafaxine, Doxepin, Duloxetine, Escitalopram, Esketamine, Fluoxetine, Fluvoxamine, Imipramine, Levomilnacipran, Mianserin, Mirtazapine, Nortriptyline, Paroxetine, Sertraline, Trazodone, Trimipramine, Vilazodone, Venlafaxine, Vortioxetine and others;
**Antipsychotics** can include Aripiprazole, Asenapine, Chlorpromazine, Clozapine, Haloperidol, Lurasidone, Olanzapine, Paliperidone, Perphenazine, Quetiapine, Risperidone, Sertindole, Sulpiride, Thioridazine, Ziprasidone, Zuclopenthixol and others;
**Stimulants** like Armodafinil, Dexamfetamine, Lisdexamfetamine, Methylphenidate, Modafinil, and others;
**Non-stimulants** like atomoxetine and others;
**Anesthetics** like Ketamine and others.

The drug list will be updated to include new molecules that may emerge in the market and are involved in the treatment of mental health disorders.

**Adjuvant agents,** any of the aforementioned types of medications can be used as adjuvant agents; even some medication not mentioned but that similarly fulfills the adjuvant function. Adjuvant agents in psychopharmacology are additional components used alongside main medications to enhance treatment effectiveness, minimize side effects, and promote overall patient well-being. Their choice and use depend on the individual needs of the patient and the specific nature of the mental disorder being treated.

The list of medications will be updated to include new molecules that may emerge on the market and be involved in the treatment of mental health disorders. For example: the release of a new antipsychotic drug based on a drug different from those currently known will still be considered an antipsychotic drug and can be easily incorporated into the list of eligible drugs through the selection method established in the present invention.

### Methodology

### STEP #1 - Determination of the attributes used for Multicriteria Decision Analysis (MCDA).

Multicriteria Decision Analysis is a useful methodology for structuring decision-making processes in healthcare. MCDA is both an approach and a set of techniques, to provide an overall ordering of options, from the most preferred to the least preferred option. The options may differ in the extent to which they achieve several objectives, and no one option will be best in achieving all objectives. In our case, medical treatment options are our options.

The attributes utilized for the analysis are gathered from Pregnancy (PR), Age Range (AR), Underlying Condition (UC), Blood Parameters (BP), Pharmacokinetics (PK), Pharmacodynamics (PD), Side Effects (SE), and Drug Interactions (DI).

Pregnancy and lactation (PR), Underlying Condition (UC), and Drug Interactions (DI) are variable attributes, meaning they are taken into account only if the patient's profile meets certain requirements. The remaining attributes are fixed and always considered. Therefore, the number of attributes considered in the multicriteria decision analysis can vary from a minimum of 5 to a maximum of 8.

Pregnancy and lactation (PR): This is a Biomarker Group 1 phenotype. This attribute is included only if the patient is a pregnant woman or is lactating. This data is obtained from the patient's self-reported information.

Age Range (AR): This is a Biomarker Group 1 phenotype. It is obtained from the patient's self-reported information. The weight assigned to this attribute and the scoring criteria for different drugs vary depending on whether the age range is Pediatric, Adult, or Geriatric.

Underlying Condition (UC): This is a Biomarker Group 1 phenotype. This attribute is included only if the patient has an underlying condition. This data is first obtained from the patient's clinical parameters reported by the treating physician. Additionally, the patient's self-reported information is also considered.

Blood Parameters (BP): These are Biomarker Group 2 phenotypes. Blood parameters data are obtained from clinical blood tests performed on the patient (complete blood count, liver function tests, thyroid profile, coagulation profile, urea, creatinine, and electrolytes, among others).

Pharmacokinetics (PK): These are Biomarker Group 3 phenotypes. This data is obtained from the genetic analysis performed on the patient (CYP1A2, CYP2D6, CYP2C9, CYP2B6, CYP2C19, CYP3A4, among others).

Pharmacodynamics (PD): These are Biomarker Group 3 parameters. This data is obtained from the genetic analysis performed on the patient extracting relevant genetic variations.

Side Effects (SE): This is a Biomarker Group 1 information. This data is obtained from self-reported questionnaires provided by the patient. The weight assigned to this attribute and the scoring criteria for different drugs vary depending on the patient's body mass index (BMI), weight changes (average kg/week), and the type of sleep disorder (Hypersomnia or Insomnia). This attribute is not considered if the patient is pregnant.

Interaction with Other Drugs (DI): This is a Biomarker Group 1 information. This attribute is included only if the patient chronically consumes any type of medication, whether psychiatric or not. This data is obtained, first, from the patient's clinical parameters reported by the medical professional in charge of the patient's treatment. Secondly, self-reported information by the patient is also considered. While this attribute does not directly affect the MCDA formula, it is taken into account to provide a final indication to the medical professional in charge of the patient's treatment regarding potential interactions of the drugs selected in the MCDA.

### STEP #2 - Determination of the weights of each attribute.

The assignment of weights to each attribute is done according to the following table calculated using the Simple Multi-Attribute Rating Technique (SMART) Edwards & Barron, 1994).

### Attribute Weights

| **UC** | **PR** | **AR** | **PK** | **BP** | **PD** | **SE** |
|---|---|---|---|---|---|---|
| | | 0,38ⁱ | 0,32 | 0,23 | 0,07 | |
| | | 0,30ⁱⁱ | 0,27 | 0,18 | 0,05 | 0,20 |
| | 0,30 | 0,20 | 0,27 | 0,18 | 0,05 | |
| 0,28 | | 0,22ⁱⁱⁱ | 0,25 | 0,18 | 0,07 | |
| 0,25 | | 0,17^{iv} | 0,22 | 0,15 | 0,05 | 0,16 |
| 0,20 | 0,30 | 0,11 | 0,20 | 0,14 | 0,05 | |

| | | | | | | |
|---|---|---|---|---|---|---|
| References: ⁱ: Pediatric/Geriatric, ⁱⁱ: Adult, ⁱⁱⁱ: Pediatric/Geriatric, ^{iv}: Adult | | | | | | |

### STEP # 3 - Scoring criteria for each attribute.

The scoring assignment depends on the analyzed pathology.

### Major Depression Disorder (MDD)

Pregnancy (PR): If The Canadian Network for Mood and Anxiety Treatments (CANMAT) recommends it as a first-line treatment, the drug is assigned a score of 100 (MacQueen et al., 2016). If recommended as a second-line treatment, the drug receives a score of 70. If recommended as a third-line treatment only under compelling circumstances, the drug is assigned a score of 40. If it is not listed in CANMAT, the drug is assigned a score of 20. If it is recommended as a third-line treatment but contraindicated for breastfeeding, the drug receives a score of 10.

Then, the recommendation of the drug is verified according to the Electronic Medicines Compendium (EMC, n.d.) or the Online Information Center for Medicines of the Spanish Agency of Medicines and Medical Devices (CIMA, n.d.). Both websites provide updated information on medicines authorized for use in the United Kingdom, which is verified and approved by either the United Kingdom Medicines and Healthcare Products Regulatory Agency (MHRA) or the European Medicines Agency (EMA). Depending on the recommendation, the scores assigned to the drug are as follows: Can be used with caution. Breastfeeding only if the benefits outweigh the risks: 100, Can be used if clinically necessary, breastfeeding should be discontinued: 80, Avoid unless strictly necessary, Breastfeeding only if the benefits outweigh the risks: 50, Avoid precautiously, breastfeeding should be discontinued: 40, Avoid precautiously. Contraindicated during breastfeeding: 30, Not listed in EMC or CIMA: 20, Avoid during pregnancy: 10.

In the third instance, according to the drug labeling by the United States Food and Drug Administration (FDA) - Pregnancy and Lactation Labeling (PPLR) (Pernia & DeMaagd, 2016), if the classification was A, B, C, D, NA (not available), or X, the drug is assigned scores of 100, 100, 80, 30, 20, or 10, respectively.

Next, the drug's breastfeeding risk category assigned by the U.S. Food and Drug Administration (FDA) is verified. According to the category, whether it is L1, L2, L3, L4, NA (unavailable), or L5, the drug is assigned scores of 100, 100, 80, 60, 20, or 10, respectively (ACOG Committee on Practice Bulletins-Obstetrics, 2008).

Finally, according to the category assigned to the drug by the categorization system of the Australian Department of Health (Therapeutic Goods Administration (TGA, n.d.), for the prescription of medications during pregnancy, whether it is A, B1, B2, B3, C, D, NA (not available), or X, the drug is assigned scores of 100, 100, 90, 80, 70, 30, 20, or 10, respectively.

The final score of the drug for the Pregnancy attribute (PR) is obtained by combining the 5 previous scores in a polynomial according to a relative weight given to each of them: CANMAT = 40, EMC/CIMA = 10, FDA = 20, FDA Lactation Risk = 10, TGA Pregnancy category = 20.

Age Range (RE): For antidepressants, scores are assigned to each drug based on the recommendations provided by various guidelines analyzed for each drug according to the patient's age range. For the adult age range, recommendations from CANMAT, the Korean Medication Algorithm Project (KMAP) (Seo et al., 2021), and The Royal Australian and New Zealand College of Psychiatrists (RANZCP) are considered (Malhi et al., 2021).

If the drug is first-line according to CANMAT and RANZCP and Treatment of Choice according to KMAP, it is assigned a score of 100. If it is first-line according to CANMAT, RAZCP, and KMAP, a score of 95 is assigned. If it is first-line according to CANMAT and KMAP, a score of 90 is assigned. If it is first-line according to CANMAT, a score of 80 is assigned. If it is second-line according to CANMAT and RAZCP, a score of 70 is assigned. If it is second-line according to CANMAT, a score of 60 is assigned. If it is third-line according to CANMAT, a score of 40 is assigned.

For the geriatric age range, the drug is assigned a score of 100, 90, 80, 70, 60, 50, 40, or 10, respectively, according to CANMAT recommendation: first-line level 1, first-line level 2, second-line level 1, second-line level 2, second-line level 2, third-line level 2, third-line level 3, or Not listed in CANMAT.

For the pediatric age range, the drug is assigned a score of 100, 80, 70, 60, 40, 20, or 10, respectively, based on the CANMAT recommendation being First-line for severe depression, Second-line for severe depression, Second-line for severe depression Level 2, Second-line for severe depression Level 3, Third-line for severe depression, Not listed in CANMAT, or Ineffective for this age group. Additionally, for the pediatric age range, the drug is also assigned a score of 100, 90, 60, 40, or 10, respectively, based on the recommendation from the American Academy of Child and Adolescent Psychiatry (AACAP) being FDA-approved for 8 years or older, FDA-approved for 12 years or older, Most commonly used SSRIs in the age group, Other antidepressants used in the age group, or No information available (Walter et al., 2023). The final score for the Pediatric Age Range attribute is obtained by combining both scores into a polynomial according to a relative weight assigned to each of them: CANMAT = 60, AACAP = 40.

For adjunctive therapies, scores are assigned to each drug based on the recommendation provided by various guidelines for each drug according to the patient's age range. For adult and geriatric age ranges, recommendations from CANMAT are considered. However, for the pediatric age range, there are no recommendations for the use of drugs as adjunctive therapies for pediatric depression, so the score is 0.

For the adult age range, if the drug is first, second, or third-line according to CANMAT, it is assigned a score of 100, 70, or 40, respectively. A score of 20 is assigned if the drug is used off-label for the condition.

For the geriatric age range, if the drug is first-line level 1, second-line level 2, or third-line level 3 according to CANMAT, it is assigned a score of 100, 90, or 70, respectively. A score of 0 is assigned if the drug is not listed in CANMAT.

Underlying Condition (UC): To assign scores to antidepressants and adjunctive therapies based on the patient's underlying condition(s), the drug recommendation is verified according to the EMC or CIMA. A score of 30 is assigned if there are publicly available studies based on scientific evidence indicating that, compared to other drugs, there is a disadvantage in using this drug in the population with this pathology, or 50 if, on the contrary, there are publicly available studies based on scientific evidence indicating that, compared to other drugs, there is an advantage in using this drug in the population with this pathology. If a drug scores 0 in this attribute, the drug is automatically eliminated from the analysis (MCDA). If there is no specific indication for that UC, a score of 100 is assigned.

Blood Parameters (BP): To assign scores to antidepressants and adjunctive therapies based on the blood parameter attribute, the drug recommendation is verified according to the EMC or CIMA. Scores of 100, 40, or 0 are assigned to the drug depending on whether the drug has no specific indication for the altered blood parameter, requires monitoring, or is contraindicated. If a drug scores 0 in this attribute, the drug is automatically eliminated from the analysis (MCDA).

Pharmacokinetics (PK): Drugs undergo different pathways of metabolism. For example CYP2D6: Amitriptyline, clomipramine, trimipramine, nortriptyline, imipramine, desipramine, doxepin, paroxetine, fluvoxamine, duloxetine, venlafaxine, vortioxetine, aripiprazole, brexpiprazole, risperidone. CYP2C19: Amitriptyline, clomipramine, trimipramine, imipramine, escitalopram, citalopram, sertraline. UGT: Desvenlafaxine. CYP2B6: Bupropion. CYP3A4: Quetiapine (CIMA, n.d.; CPIC, n.d., Flockhart et al., 2021).

To assign scores to antidepressants and adjunctive agents based on the patient's genetic phenotype, each drug is scored according to the following criteria:
100 if the metabolism pathway (CYP450 enzymes or UGT) is normal. 90 if caution is required because it is inferred that the metabolism pathway may be altered. It is advised to start treatment with the recommended initial dose and use therapeutic monitoring for dose adjustments. Specific recommendations exist in the CPIC. 80 if caution is required because it is inferred that the metabolism pathway may be altered. It is advised to start treatment with the recommended initial dose and use therapeutic monitoring for dose adjustments. There are no specific recommendations in the CPIC. 60 if caution is required because it is inferred that the metabolism pathway may be altered. It is advised to consider an alternative drug. If the drug is justified, therapeutic monitoring is suggested for dose adjustments. Specific recommendations exist in the CPIC. 50 if caution is required because it is inferred that the metabolism pathway may be altered. It is advised to consider an alternative drug. If the drug is justified, it is suggested to follow the specific recommendations of the CPIC for dose adjustment. 20 if caution is required because it is inferred that the metabolism pathway may be altered. It is advised to consider an alternative drug. Specific recommendations exist in the CPIC for dose adjustment.

Side Effects (SE): To assign scores to antidepressants and adjunctive agents, the following factors are considered: a) Clinical parameter Weight Change (increase, none, or loss), b) Body Mass Index (Underweight, normal weight, overweight, or obesity), and c) Sleep Disorder parameter (Insomnia, none, or hypersomnia) according to available information EMC, CIMA, and CANMAT (CIMA, n.d.; Dent et al., 2012; Goldberg & Ernst, 2019; MacQueen et al., 2016; Manning & Blumenthal, 2023; Eugene, 2020; Schatzberg et al., 2019). Based on the combination of these three patient parameters, scores are assigned to each drug, considering their side effects in terms of drowsiness and body weight. This attribute does not apply to pregnant women and pediatric or geriatric age ranges.

Pharmacodynamics (PD): According to their effect, the phenotype of genetic variations influencing pharmacodynamics can be classified into two categories: Those affecting the drug's efficacy and those increasing the risk of toxicity (adverse effects) of the drug (Gong et al., 2021; Whirl-Carrillo et al., 2012; Whirl-Carrillo et al., 2021). Each antidepressant or adjunctive agent is assigned a score of 10, 5, or 0, depending on whether the genetic variation is favorable, neutral, or unfavorable. By adding up the scores for each genetic variation, a subtotal is calculated for the Efficacy category and another for Toxicity. Then, for each of the categories, each drug is assigned 50, 37, 25, 12, 5, or 0 points, depending on whether it obtained 100% of the available points, More than 50%, 50%, Less than 50%, No points available, or none of the available points. Finally, the final score for the drug is assigned by adding up the points obtained in each category.

Drug Interaction (IF): Evaluating the information available in DrugBank Knowledgebase (Knox et al., 2024) and Drug Interactions Flockhart Table (Flockhart et al., 2021), a score of 100, 80, 60, 40, 20, or 0 is assigned to the drug (antidepressant or adjunctive agent), respectively, if there is no specific indication, if there is a mild interaction, if caution is required, if supervision is required, if alternatives need to be sought, or if the drug is contraindicated.

### Bipolar Disorder (BPD)

Pregnancy (PR): As in MDD, the recommendation of the drug is verified according to the EMC or CIMA, PPLR, FDA breastfeeding risk category, and TGA categorization system for the prescription of medications during pregnancy.

Age Range (RE): In bipolar depression, score calculation and/or the group of drugs are different according to the current episode diagnosed by the clinician.
1. BPD in a current depressive episode:
   The medication considered for treatment of this diagnosis include Quetiapine, Lurasidone (+Lithium/Valproic Acid), Lurasidone, Lithium, Valproic Acid, Lamotrigine, Lamotrigine (Adjunctive), Bupropion (Adjunctive), Citalopram (Adjunctive), Escitalopram (Adjunctive), Fluoxetine (Adjunctive), Fluvoxamine (Adjunctive), Paroxetine (Adjunctive), Sertraline (Adjunctive), Cariprazine, Olanzapine (+Fluoxetine), Aripiprazole (Adjunctive), Armodafinil (Adjunctive), Carbamazepine, Ketamine IV (Adjunctive), Levothyroxine (Adjunctive), Modafinil (Adjunctive), Olanzapine, Pramipexole (Adjunctive), Asenapine (Adjunctive).
   Scores are assigned to each drug based on the recommendations provided by CANMAT (Yatham et al., 2018) and AACAP (Cox et al., 2014) according to the patient's age range. For the adult and geriatric age range, recommendations from CANMAT are considered. If the drug is first-line according to CANMAT, it is assigned a score of 100. If it is second-line according to CANMAT, a score of 50 is assigned. If it is third-line according to CANMAT, a score of 30 is assigned, if it is not listed for the age range a 10 is assigned.
   For the pediatric age range, the drug is assigned a score of 100, 50, 30, and 10, respectively, based on the CANMAT recommendation being First-line, Second-line, and Third-line, Not listed in CANMAT. Additionally, for the pediatric age range, the drug is also assigned a score of 100, 90, 60, 40, or 10, respectively, based on the recommendation from the AACAP being FDA-approved for 10 years or older, FDA-approved for 12 years or older, FDA-approved for 13 years or older, Other antidepressants used in the age group, or No information available. The final score for the Pediatric Age Range attribute is obtained by combining both scores into a polynomial according to a relative weight assigned to each of them: CANMAT = 60, AACAP = 40.
2. BPD in a current Hypomanic or manic episode:
   The medication considered for treatment of this diagnosis include Quetiapine, Quetiapine (+ Lithium/Valproic Acid), Lithium, Lithium + Valproic Acid, Valproic Acid, Clonazepam, Carbamazepine (+ Lithium/Valproic Acid), Cariprazine, Olanzapine (+ Lithium/Valproic Acid), Aripiprazole, Aripiprazole (+ Lithium/Valproic Acid), Carbamazepine, Oxcarbazepine (+ Lithium/Valproic Acid), Clozapine, Haloperidol (+ Lithium/Valproic Acid), Haloperidol, Olanzapine, Ziprasidone, Asenapine, Asenapine (+ Lithium/Valproic Acid), Paliperidone, Risperidone, Risperidone (+ Lithium/Valproic Acid).
   Scores are assigned to each drug for each age range in the same way as for BPD in a current depressive episode.
3. BPD in a current depressive episode with mixed features:
   The medication considered for treatment of this diagnosis include Quetiapine, Lurasidone, Lithium, Valproic Acid, Lamotrigine, Cariprazine, Olanzapine (+ Fluoxetine), Olanzapine, Asenapine, Ziprasidone.
   Similarly, scores are assigned to each drug for each age range according to the same criteria and procedures as for BPD in a current depressive episode.
4. BPD in a current Hypomanic or manic episode with mixed features:
   The medication considered for treatment of this diagnosis include Quetiapine, Lithium, Valproic Acid, Cariprazine, Olanzapine (+ Lithium/Valproic Acid), Aripiprazole, Carbamazepine, Olanzapine, Ziprasidone, Lurasidone, Asenapine, Risperidone. Scores are assigned to each drug for each age range according to the same criteria and procedures as for BPD in a current depressive episode except that the final score for the Pediatric Age Range attribute is obtained by combining both scores into a polynomial according to a relative weight assigned to each of them: CANMAT = 70, AACAP = 30.

Underlying Condition (UC): The medications considered include Bupropion, Citalopram, Escitalopram, Fluoxetine, Fluvoxamine, Paroxetine, Sertraline, Cariprazine, Pramipexole, Modafinil, Levothyroxine, Ketamine, Armodafinil, Aripiprazole, Lithium, Lurasidone, Olanzapine, Quetiapine, Valproic acid, Lamotrigine, Carbamazepine, Oxcarbazepine, Asenapine, Paliperidone, Risperidone, Ziprasidone, Haloperidol, Clozapine, Clonazepam. To assign scores to medications based on the patient's underlying condition(s), the drug recommendation is verified according to the EMC or CIMA with the same rationale and scoring procedures as those detailed above for MDD.

Blood Parameters (BP): The medications considered are the same as listed above for UC. To assign scores to medications based on the patient's blood parameters, the drug recommendation is verified according to the EMC or CIMA with the same rationale and scoring procedures as those detailed above for PB in MDD.

Pharmacokinetics (PK): The medications considered are the same as those for UC, EM, and PB. To assign scores to medications based on the patient's pharmacokinetics, the same rationale and scoring procedures as those detailed above for PK in MDD.

Side Effects (SE): The medications considered are the same as those for UC, EM, PB and PK. To assign scores to medications based on side effects, the same rationale and scoring procedures are detailed above for SE in MDD. Pharmacodynamics (PD): The medications considered are the same as those for UC, EM, PB, and SE. To assign scores to medications based on the patient's pharmacokinetics, the same rationale and scoring procedures as those detailed above for PD in MDD.

Drug Interaction (IF): The medications considered are the same as those for UC, EM, PB, SE, and PD. To assign scores to medications based on drug interactions, the same rationale and scoring procedures as those detailed above for IF in MDD.

### General Anxiety Disorder (GAD)

The medications considered can include Escitalopram, Paroxetine, Fluvoxamine, Sertraline, Venlafaxine, Duloxetine, Agomelatine, Imipramine, Pregabalin, Vilazodone, Alprazolam, Bromazepam, Diazepam, Lorazepam, Hydroxyzine, Quetiapine, Bupropion, Buspirone, Vortioxetine, Risperidone, Olanzapine, Valproic Acid, Fluoxetine, Mirtazapine, Trazodone, Citalopram

Pregnancy (PR): Given the relatively large database regarding their safety in the perinatal period, Selective Serotonin Reuptake Inhibitors (SSRIs), Citalopram, Escitalopram, Fluoxetine, Fluvoxamine, Paroxetine, Sertraline, are also the first line treatments for these anxiety disorders pre-conception and antenatally are first-line for GAD according to British Association for Psychopharmacology (BAP) (McAllister-Williams et al., 2017). To assign scores to medications, the recommendation of the drug is verified according to the EMC or CIMA, PPLR, FDA breastfeeding risk category, and TGA categorization system for the prescription of medications during pregnancy with the same rationale and scoring procedures as those detailed above for MDD and BPD.

Age Range (RE): A range of effective treatments for GAD are available, particularly duloxetine, escitalopram, pregabalin, quetiapine, and venlafaxine (Fagan & Baldwin, 2023). Scores are assigned to each drug based on the recommendations provided by various guidelines analyzed for each drug according to the patient's age range. For the adult age range, recommendations from CANMAT (Katzman et al., 2014), the World Federation of Societies of Biological Psychiatry (WFSBP) (Bandelow et al., 2023), and the BAP (Baldwin et al., 2014).

For the Adult and geriatric age ranges, the drug is assigned a score of 100, 80, 60, 40, or 10, respectively, based on the WFSBP recommendation being First-line for GAD, Second-line, Third-line (with Strong evidence for the intervention) or Third-line (with Limited evidence for the intervention), Not listed mentioned in previous groups or not mentioned in WFSBP respectively. Additionally, for the Adult and Geriatric age ranges, the drug is also assigned a score of 100, 80, or 20, for First-line, Second-line, or No recommendation according to BAP. Finally, the medication is assigned a score of 100, 80, 60, or 20, for First-line, Second-line, Third-line, or No recommendation according to the BAP final score for the Adult and geriatric Age Range attribute is obtained by combining both scores into a polynomial according to a relative weight assigned to each of them: WFSBP = 80, BAP = 5, CANMAT = 15.

For the pediatric age range, psychological treatments are preferred. A limited number of Randomized Controlled Trials (RCTs) support the efficacy of SSRIs in this group, and current guidelines recommend using SSRIs as a first-line pharmacological treatment (Bandelow et al., 2023; Bobbitt et al., 2023; Fagan & Baldwin, 2023; Katzman et al., 2014; Strawn et al., 2018). The drug is assigned a score of 100, 80, or 20, respectively, based on the WFSBP recommendation being First-line (with Strong evidence for the intervention) for GAD, Second-line (with Limited evidence for the intervention) for GAD, Not listed mentioned in previous groups or not mentioned in WFSBP respectively.

Underlying Condition (UC): To assign scores to medications based on the patient's underlying condition(s), the drug recommendation is verified according to the EMC or CIMA with the same rationale and scoring procedures as those detailed above for MDD and BPD.

Blood Parameters (BP): To assign medication scores based on the patient's blood parameters, the drug recommendation is verified according to the EMC or CIMA with the same rationale and scoring procedures as those detailed above for PB in MDD and BPD.

Pharmacokinetics (PK): To assign scores to medications based on the patient's pharmacokinetics, the same rationale and scoring procedures as those detailed above for PK in MDD and BPD.

Side Effects (SE): To assign scores to medications based on side effects, the same rationale and scoring procedures as those detailed above for SE in MDD.

Pharmacodynamics (PD): To assign scores to medications based on the patient's pharmacodynamics, the same rationale and scoring procedures as those detailed above for PD in MDD.

Drug Interaction (IF): To assign scores to medications based on drug interactions, the same rationale and scoring procedures as those detailed above for IF in MDD.

### Social Anxiety Disorder (SAD)

The medications considered can include Escitalopram, Paroxetine, Fluvoxamine, Sertraline, Venlafaxine, Pregabalin, Phenelzine, Citalopram, Bromazepam, Clonazepam, Moclobemide, Mirtazapine, Gabapentin, Ketamine, Fluoxetine, Buspirone, Mirtazapine (Baldwin et al., 2014; Bandelow et al., 2023; Katzman et al., 2014).

Pregnancy (PR): As GAD, SSRIs, are also the first line treatments for SAD according to BAP (McAllister-Williams et al., 2017). As GAD, to assign scores to medications based, the recommendation of the drug is verified according to the EMC or CIMA, PPLR, FDA breastfeeding risk category, and TGA categorization system for the prescription of medications during pregnancy with the same rationale and scoring procedures as those detailed above for MDD and BPD.

Age Range (RE): Scores are assigned to each drug based on the recommendations provided by various guidelines analyzed for each drug according to the patient's age range. As in GAD, recommendations from CANMAT, WFSBP, and BAP for the adult age range (Baldwin et al., 2014; Bandelow et al., 2023; Katzman et al., 2014).

For the Adult and geriatric age ranges, the drug is assigned a score of 100, 80, 60, 40, or 10, respectively, based on the WFSBP recommendation being First-line for SAD, Second-line (with Strong evidence for the intervention), Second-line (with Limited evidence for the intervention), Third-line (with Limited evidence for the intervention), Not listed mentioned in previous groups or not mentioned in WFSBP respectively. Additionally, for the Adult and Geriatric age ranges, the drug is also assigned a score of 100, 80, or 20, for First-line, Second-line, or No recommendation according to BAP. Finally, the medication is assigned a score of 100, 80, 60, or 20, for First-line, Second-line, Third-line, or No recommendation according to the BAP final score for the Adult and geriatric Age Range attribute is obtained by combining both scores into a polynomial according to a relative weight assigned to each of them: WFSBP = 80, BAP = 5, CANMAT = 15.

For the pediatric age range, psychological treatments are preferred. A limited number of RCTs support the efficacy of SSRIs in this group, and current guidelines recommend using SSRIs as a first-line pharmacological treatment (Baldwin et al., 2014; Bandelow et al., 2023; Bobbitt et al., 2023; Katzman et al., 2014). The drug is assigned a score of 100, 80, or 20, respectively, based on the WFSBP recommendation being Second-line (with Weak evidence for the intervention) for SAD, Third-line (with Limited evidence for the intervention) for SAD, Not listed mentioned in previous groups or not mentioned in WFSBP respectively.

Underlying Condition (UC): To assign scores to medications based on the patient's underlying condition(s), the drug recommendation is verified according to the EMC or CIMA with the same rationale and scoring procedures as those detailed above for MDD, BPD, and GAD.

Blood Parameters (BP): To assign medication scores based on the patient's blood parameters, the drug recommendation is verified according to the EMC or CIMA with the same rationale and scoring procedures as those detailed above for BP in MDD, BPD, and GAD.

Pharmacokinetics (PK): To assign scores to medications based on the patient's pharmacokinetics, the same rationale and scoring procedures as those detailed above for PK in MDD and BPD.

Side Effects (SE): To assign scores to medications based on drug side effects, the same rationale and scoring procedures as those detailed above for SE in MDD.

Pharmacodynamics (PD): To assign scores to medications based on the patient's pharmacodynamics, the same rationale and scoring procedures as those detailed above for PD in MDD.

Drug Interaction (IF): To assign scores to medications based on drug interactions, the same rationale and scoring procedures as those detailed above for IF in MDD.

### Psychosis (PS):

The medications considered include Chlorpromazine, Haloperidol, Aripiprazole, Asenapine, Clozapine, Iloperidone, Lurasidone, Olanzapine, Paliperidone, Quetiapine, Risperidone, Ziprasidone (Keepers et al., 2020).

Pregnancy (PR): Haloperidol, Risperidone, Olanzapine, Quetiapine, and Chlorpromazine are listed in the recommendations for the use of antipsychotics in pregnancy and lactation by the WBSFP (Hasan et al., 2015). To assign scores to medications, the recommendation of the drug is verified according to the WBSFP, PPLR, FDA breastfeeding risk category, and TGA categorization system for the prescription of medications during pregnancy.

If WBSFP recommends it with a safety rating of 4 for both pregnancy and breastfeeding, the drug is assigned a score of 100, if recommended with a safety rating of 4 for pregnancy is assigned a score of 80 and is assigned a score of 60 if recommended with a safety rating of 4 for breastfeeding.

Secondly, according to PPLR, if the classification was A, B, C, D, NA (not available), or X, the drug is assigned scores of 100, 100, 80, 30, 20, or 10, respectively. Next, the drug's breastfeeding risk category assigned by the U.S. Food and Drug Administration (FDA) is verified. According to the category, whether it is L1, L2, L3, L4, NA (unavailable), or L5, the drug is assigned scores of 100, 100, 80, 60, 20, or 10, respectively (ACOG Committee on Practice Bulletins-Obstetrics, 2008).

Finally, according to the category assigned to the drug by the categorization system of the TGA, for the prescription of medications during pregnancy, whether it is A, B1, B2, B3, C, D, NA (not available), or X, the drug is assigned scores of 100, 100, 90, 80, 70, 30, 20, or 10, respectively (TGA, n.d.).

The final score of the drug for the Pregnancy attribute (PR) is obtained by combining the 4 previous scores in a polynomial according to a relative weight given to each of them: WBSFP = 75, FDA PPLR = 5, FDA PPLR LR = 5, TGA PC = 15.

Age Range (RE): Scores are assigned to each drug based on the recommendations provided by various guidelines analyzed for each drug according to the patient's age range.

For the Adult age range, score calculation and/or the group of drugs are different according to the first episode or multi-episode as defined by the clinician (Hasan et al., 2017). For the first episode, the drug is assigned a score of 100, 80, 60, 30, or 10, respectively, based on the WFSBP recommendation being First-line (with Strong evidence for the intervention), Second-line (with Strong evidence for the intervention), Second-line (with Limited evidence for the intervention), Lack of evidence or Not listed in previous groups or not mentioned in WFSBP respectively (Hasan et al., 2017). Additionally, for the Adult age range first-episode, the drug is also assigned a score of 100 or 10, for First-line or No recommendation according to RANCZP (Galletly et al., 2016). Age Range attribute is obtained by combining both scores into a polynomial according to a relative weight assigned to each of them: WFSBP = 70, RANCZP = 30.

For the Geriatric age range, score calculation is done considering a higher score for those antipsychotics most used on older people. (Korkatti-Puoskari et al. 2023; Ng et al., 2023). The drug is assigned a score of 100, or 10, respectively.

For the pediatric age range, although considerable progress has been made in furthering schizophrenia treatment in children and adolescents, there is a clear need for more robust studies to better differentiate between available options. When selecting an antipsychotic medication in practice, those with FDA approval should generally be considered first (Lee et al., 2020). The drug is assigned a score of 100 or 10, respectively, based on the FDA approval for pediatric use.

Underlying Condition (UC): To assign scores to medications based on the patient's underlying condition(s), the drug recommendation is verified according to the EMC or CIMA with the same rationale and scoring procedures as those detailed above for MDD, BPD, GAD, and SAD.

Blood Parameters (BP): To assign medication scores based on the patient's blood parameters, the drug recommendation is verified according to the EMC or CIMA with the same rationale and scoring procedures as those detailed above for BP in MDD, BPD, GAD, and SAD.

Pharmacokinetics (PK): To assign scores to medications based on the patient's pharmacokinetics, the same rationale and scoring procedures as those detailed above for PK in MDD and BPD.

Side Effects (SE): As done previously for MDD, BP, GAD, and SAD, to assign scores to drugs, the following factors are considered: a) Clinical parameter Weight Change (increase, none, or loss), b) Body Mass Index (Underweight, normal weight, overweight, or obesity), and c) Sleep Disorder parameter (Insomnia, none, or hypersomnia) according to available information EMC (EMC, n.d.), CIMA (CIMA, n.d.), and CANMAT (MacQueen et al., 2016). Based on the combination of these three patient parameters, scores are assigned to each drug, considering their side effects in terms of drowsiness and body weight.

Additionally, the side effects of akathisia, Parkinsonism, Dystonia, Tardive dyskinesia, Hyperprolactinemia, and Anticholinergic effects were considered (Keepers et al., 2020). For each adverse effect that the patient reported on previous medication (See Biomarker Group #1), the score assigned to the drug is multiplied by a factor according to the following table.

| | Akathisia | Parkinsonism | Dystonis | Tardive dyskinesia | Hyperprolactinemia | Anticholinergic Effects |
|---|---|---|---|---|---|---|
| Amisulpride | 1 | 0.5 | 0.5 | 0.5 | 1 | 0.5 |
| Aripiprazole | 0.5 | 1 | 0.5 | 1 | 1 | 1 |
| Asenapine | 0.5 | 1 | 1 | 0.5 | 0.5 | 1 |
| Clozapine | 1 | 1 | 1 | 1 | 1 | 0.2 |
| Haloperidol | 0.2 | 0.2 | 0.2 | 0.2 | 0.2 | 1 |
| Iloperidone | 1 | 1 | 1 | 1 | 0.5 | 1 |
| Lurasidone | 0.5 | 0.5 | 0.5 | 0.5 | 1 | 1 |
| Olanzapine | 0.5 | 0.5 | 1 | 1 | 0.5 | 0.5 |
| Paliperidone | 0.5 | 0.5 | 0.5 | 0.5 | 0.5 | 1 |
| Quetiapine | 1 | 1 | 1 | 1 | 1 | 0.5 |
| Risperidone | 0.5 | 0.5 | 0.5 | 0.5 | 0.5 | 1 |
| Ziprasidone | 0.5 | 1 | 1 | 1 | 0.5 | 1 |
| Chlorpromazine | 0.5 | 0.5 | 0.5 | 0.2 | 1 | 0.2 |

For example, if the score assigned to Risperidone according to the Clinical parameter Weight Change, Body Mass Index, and Sleep Disorder parameter is 80, and the patient has reported Dystonia and Parkinsonism as adverse side effects of previous medications, then the final score assigned is 20 (80x0.5x0.5). This attribute does not apply to pregnant women and pediatric or geriatric age ranges.

Pharmacodynamics (PD): To assign scores to medications based on the patient's pharmacodynamics, the same rationale and scoring procedures as those detailed above for BP in MDD, BPD, GAD, and SAD.

Drug Interaction (IF): To assign scores to medications based on drug interactions, the same rationale and scoring procedures as those detailed above for PK in MDD and BPD.

### Attention Deficit Disorder (ADD):

The medications considered include Methylphenidate, Lisdexamfetamine, Dexamphetamine, Atomoxetine, Bupropion, and Modafinil (National Institute for Health and Care Excellence [NICE], 2019; Royal College of Psychiatrists, 2023).

Pregnancy (PR): The available data are too limited to make an unequivocal recommendation regarding the use of ADHD medication during pregnancy (Li et al., 2020; McAllister-Williams et al., 2017). As suggested by the BAP and Royal College of Psychiatrists (RCPsych), methylphenidate has more studies regarding its use during pregnancy (Royal College of Psychiatrists, 2023). The recommendation of the drug is verified according to the BAP, Hale's Manual of Lactational Pharmacology (HMLP) (Hale, 2020), FDA breastfeeding risk category, and TGA categorization system for the prescription of medications during pregnancy.

If BAP recommends it for both pregnancy and breastfeeding, the drug is assigned a score of 100, if there is a lack of evidence for its use for pregnancy is assigned a score of 50 and is assigned a score of 20 if not listed.

Secondly, according to PPLR, if the classification was A, B, C, D, NA (not available), or X, the drug is assigned scores of 100, 100, 80, 30, 20, or 10, respectively.

Thirdly, according to HMLP, if the classification was L2 (Drug has been studied in a limited number of breastfeeding women without evidence of adverse effects in nursing infants.), L3 (Studies in breastfeeding have shown evidence for mild non-threatening adverse effects OR there are no studies in breastfeeding for a drug with possible adverse effects) or L4 (Studies have shown evidence for risk to a nursing infant, but in some circumstances, the drug may be used during breastfeeding), the drug is assigned scores of 100, 80, 30, respectively.

Finally, according to the category assigned to the drug by the categorization system of the TGA, for the prescription of medications during pregnancy, whether it is A, B1, B2, B3, C, D, NA (not available), or X, the drug is assigned scores of 100, 100, 90, 80, 70, 30, 20, or 10, respectively (TGA, n.d.).

The final score of the drug for the Pregnancy attribute (PR) is obtained by combining the 3 previous scores in a polynomial according to a relative weight given to each of them: BAP = 20, FDA PPLR = 20, HMLP = 30, TGA PC = 30.

Age Range (RE): For the Adult and geriatric age range, the drug is assigned a score of 100 or 60, respectively, based on the RCPsych recommendation being the First-line or Second-line treatment option.

For the pediatric age range, recommendations from international clinical guidelines are inconsistent concerning the order in which the stimulants should be offered with some guidelines advocating for methylphenidate over amphetamines in children while other guidelines make no distinction between the stimulants (Chan et al., 2023; Cortese et al., 2018; Kooij et al., 2010). We rated methylphenidate over amphetamines in children and gave a lower score to non-stimulants (Wolraich, 2019).

Underlying Condition (UC): To assign scores to medications based on the patient's underlying condition(s), the drug recommendation is verified according to the EMC or CIMA with the same rationale and scoring procedures as those detailed above for MDD, BPD, GAD, SAD, and PS.

Blood Parameters (BP): To assign medication scores based on the patient's blood parameters, the drug recommendation is verified according to the EMC or CIMA with the same rationale and scoring procedures as those detailed above for BP, MDD, BPD, GAD, SAD, and PS.

Pharmacokinetics (PK): To assign scores to medications based on the patient's pharmacokinetics, the same rationale and scoring procedures as those detailed above for BP, MDD, BPD, GAD, SAD, and PS.

Side Effects (SE): To assign scores to medications based on the patient's pharmacokinetics, the same rationale and scoring procedures as those detailed above for BP, MDD, BPD, GAD, SAD, and PS.

Pharmacodynamics (PD): To assign scores to medications based on the patient's pharmacokinetics, the same rationale and scoring procedures as those detailed above for BP, MDD, BPD, GAD, SAD, and PS.

Drug Interaction (IF): To assign scores to medications based on the patient's pharmacokinetics, the same rationale, and scoring procedures as those detailed above for BP, MDD, BPD, GAD, SAD, and PS.

### Examples

Patients diagnosed with unipolar depression were recruited and divided into two groups. The first group Treatment as Usual (TAU) received treatment according to psychiatry clinical guidelines. The second group received Clinical Decision Support System Guided (CCDSg) additional support from the method for selecting neuropsychiatric drugs. Follow-up was conducted using the SIGH-D-17 scale in both groups at the beginning of treatment and 8 and 12 weeks.

### Results

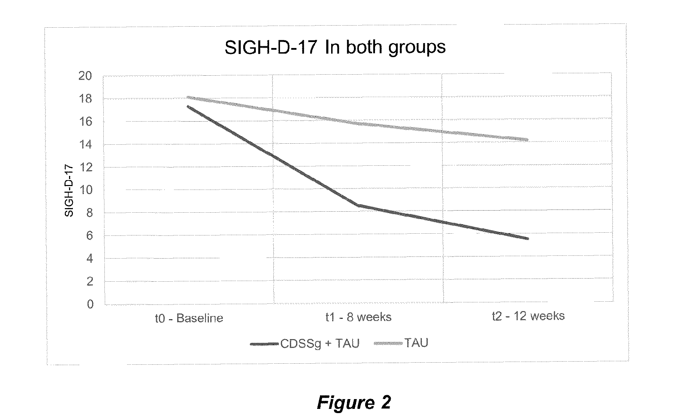

A preliminary analysis of the results was conducted with the first 20 patients. A significant interaction effect was found in the repeated measures ANOVA test (F = 13.44, p = .003, eta2 = .490). Patients whose psychiatrists received the result of the method for selecting drugs at the beginning of treatment showed fewer reported signs and symptoms of depression (at both 8 and 12 weeks) compared to those who received it later. See figure 2.

Here are four detailed examples from the study.

### Example 1

A 32-year-old male diagnosed with severe unipolar depression. He exhibits a subtherapeutic response to a treatment regimen consisting of escitalopram, sertraline, risperidone, clonazepam, and lamotrigine. Additionally, he reports taking diclofenac. Clinical parameters indicate a Body Mass Index (BMI) corresponding to Grade II overweight, with no recent weight gain. He experiences hypersomnia but no other comorbidities with depression.

Blood parameters reveal hypercholesterolemia and hypertriglyceridemia. Genetically, he exhibits increased metabolism for CYP2C19, CYP1A2, and UGT1A4 in pharmacokinetics, while enzyme activity is reduced in CYP2C9.

Considering both genetic variables and biomarkers, the top choices for antidepressant treatment are venlafaxine, desvenlafaxine, fluoxetine, bupropion, and vortioxetine. Among the adjunctive agents, aripiprazole, risperidone, quetiapine, lithium, and brexpiprazole scored the highest based on similar considerations.

Based on the algorithm results, it was decided to discontinue the previous pharmacological regimen and initiate a new one consisting of desvenlafaxine and aripiprazole. A significant response was observed within 8 weeks of treatment initiation (69% reduction in SIGH-D-17 scale).

### Detail:

1. Collection of clinical parameters (Data reported by the medical professional and by the patient).

For the source of obtaining the value of each parameter, see the section "Biomarker Group 1 - Phenotypes - Clinical Parameters" above.

Patient ID: 9. Calculation: consecutive number assigned by the system.

Gender: Male. Calculation: The patient selects from the dropdown menu.

Date of birth: April 5, 1990. Calculation: The patient selects from the calendar menu.

Age: 32. Calculation: calculated by the system.

Pregnancy: No. Calculation: automatically determined by gender.

Body Mass Index (BMI): Grade II overweight (pre-obesity). Calculation: assigned by the system as the height/weight^2 is between 27 and 29.9.

Attribute Age range (AR): Adult, Sleep disorder: Hypersomnia, Weight change: No, Smoker: Yes, Bipolar Spectrum: No, Depression Severity: Severe, Underlying Pathology: None, Current Medications: Clonazepam, Diclofenac, Phenytoin, Escitalopram, Lamotrigine, Risperidone, Sertraline.

2. Collection of blood parameters (Data obtained from the patient's blood sample).

For the source of obtaining the value of each parameter, see the "Biomarker Group 2 - Phenotypes - Blood Parameters" section above).

It is obtained from the blood analysis:
White blood cell count values: Normal, neutrophils: Normal, eosinophils: Normal, basophils: Normal, lymphocytes: Normal, monocytes: Normal, blood glucose: Normal, urea: Normal, creatinine: Normal, total cholesterol: Elevated, TGO: Normal, TGO: Normal, TGP: Normal, bilirubin: Normal, renal dysfunction: No, renal failure: No, hepatic dysfunction: No, hepatic failure: No, Anemia: No, Hyperthyroidism: No, Hypothyroidism: No, Hyponatremia: No.

Leukopenia: No, hemoglobin: Normal, hematocrit: Normal, alkaline phosphatase: Normal, proteins: Normal, albumin: Normal, TSH: Elevated, T3: Normal, T4L: Normal, triglycerides: Elevated, HDL cholesterol: Decreased, LDL cholesterol: Elevated, platelet count: Normal, prothrombin concentration: Normal, INR: Normal, partial thromboplastin time: Normal, sodium level: Normal, potassium level: Normal.

3. Compilation of genetic parameters (Data obtained from the patient's DNA sample).

For the source of obtaining the value of each parameter, see the section "Biomarker Group 3 - Genotypes - Blood Parameters" above.

Among the genetic variations, the rs762551-AC polymorphism is found in the gene and represents a rapid metabolizer CYP1A2*1A/1F. Additionally, the genetic variations rs1065852-AG and rs1135840-CG indicate a normal metabolizer status for CYP2D6 *1/*10. On the other hand, normal metabolism is observed for CYP2B6 *1/*1, CYP3A4 *1/*1, and UGT 1A1 *1/*1. Metabolism via CYP2C9 *1/*2 is intermediate (rs1799853-CT). The enzymatic activity of UGT1A4 is increased (UGT1A4, rs2011425-GT), and likewise, the CYP2C19 *1/*17 metabolism pathway is affected by the genetic variation rs12248560-CT.

In addition to the genetic variations in cytochrome P450 and UGT genes, the panel used for the patient includes 157 genetic variations that generate annotations available in PharmGKB for the following drugs: Agomelatine, Bupropion, Citalopram, Escitalopram, Fluoxetine, Fluvoxamine, Paroxetine, Sertraline, Desvenlafaxine, Duloxetine, Venlafaxine, Mirtazapine, Mianserin, Vortioxetine, Amitriptyline, Nortriptyline, Clomipramine, Desipramine, Doxepin, Imipramine, Trimipramine, Quetiapine, Aripiprazole, Ziprasidone, Lurasidone, Cariprazine, Trazodone, Tranylcypromine, Vilazodone, Risperidone, Brexpiprazole, Modafinil, Methylphenidate, Lisdexamfetamine, Armodafinil, Olanzapine, Triiodothyronine, Ketamine, Lithium, and Esketamine. PharmGKB annotations report the association between a genetic variant and a drug phenotype from a single publication (Gong et al., 2021).

4. First step of the Multicriteria Decision Analysis (MCDA) methodology.

Determination of the attributes used in MCDA. Considering that the person is male and does not present underlying pathologies, according to the considerations for determining the attributes used in MCDA described earlier (see Methodology Step # 1), the attributes for analysis are Age Range (AR), Blood Parameters (BP), Pharmacokinetics (PK), Pharmacodynamics (PD), and Side Effects (SE).

5. Second step of the MCDA methodology

Determination of the weights of each attribute. Considering again that the person is male and does not have underlying pathologies, according to the table in Methodology Step #2, we obtain the following weights for the attributes: AR: 0.30, PK: 0.27, PB: 0.18, PD: 0.05, and SE: 0.20.

6. Third step of the MCDA methodology.

Scoring criteria for each attribute. Considering the criteria established for assigning scores to each attribute (see Methodology Step #3), they are assigned for each drug. For example, if we take two drugs, venlafaxine, and aripiprazole, their scores are assigned as follows:
AR - Age Range: Venlafaxine is an antidepressant suggested as first-line treatment for unipolar depression by CANMAT and RANCZP clinical guidelines. It is also the treatment of choice according to KMAP, consequently, it is assigned a score of 100. Aripiprazole is an atypical antipsychotic suggested as a first-line adjunctive treatment for unipolar depression by CANMAT clinical guidelines, it is assigned a score of 100.
SE - Side Effects: For algorithm analysis, the body mass index, weight changes secondary to depressive episodes, and sleep disturbances are considered. The patient is Grade II overweight, has no weight change secondary to the depressive episode, and sleep disorder (hypersomnia). When evaluating the use of venlafaxine from the perspective of sleep and eating disturbance, according to available information, it is considered a drug with minimal effect on weight gain and moderate somnolence. When evaluating the use of aripiprazole from the perspective of sleep and eating disturbance, according to available information, it is considered a drug with a weight gain effect and moderate somnolence. The ranking number on which the drug is evaluated is obtained from the following tables:

### Antidepressants:

### BMI

| | | Underweight | Normal weight | Overweight | Obesity | | |
|---|---|---|---|---|---|---|---|
| | Loss | 7 | 8 | 9 | 9 | Insomnia | |
| | | 1 | 2 | 6 | 6 | None | |
| **WEIGHT CHANGE** | | 10 | 11 | 12 | 12 | Hypersomnia | **Sleep disorder** |
| | None | 7 | 13 | 15 | 17 | Insomnia | |
| | | 1 | 3 | 4 | 5 | None | |
| | | 10 | 14 | 16 | 18 | Hypersomnia | |
| | Gain | 8 | 15 | 17 | 17 | Insomnia | |
| | | | | | | | |

| | | | | | | | |
|---|---|---|---|---|---|---|---|
| | 2 | | 4 | 5 | 5 | | None Hypersomnia |
| | 11 | | 16 | 18 | 18 | | |

### Adjunctive agents:

### BMI

| | | Underweight | Normal weight | Overweight | Obesity | | |
|---|---|---|---|---|---|---|---|
| | Loss | 11 | 9 | 9 | 10 | Insomnia | |
| | | 8 | 7 | 1 | 1 | None | |
| **WEIGHT CHANGE** | | 12 | 12 | 5 | 12 | Hypersomnia | Sleep disorder |
| | None | 3 | 2 | 2 | 4 | Insomnia | |
| | | 1 | 1 | 1 | 1 | None | |
| | | 5 | 5 | 5 | 6 | Hypersomnia | |
| | Gain | 3 | 15 | 15 | 16 | Insomnia | |
| | | 1 | 13 | 13 | 14 | None | |
| | | 5 | 17 | 17 | 18 | Hypersomnia | |

The rankings obtained are number 16 for antidepressants and number 5 for adjunctive agents.

| Ranking Number | 16 | Ranking Number 5 | |
|---|---|---|---|
| Antidepressive | Score | Adjunctive agent | Score |
| Bupropion | 100 | Aripiprazole | 100 |
| Fluoxetine | 100 | Quetiapine | 60 |
| Venlafaxine | 100 | Risperidone | 100 |
| Fluvoxamine | 100 | Brexpiprazole | 100 |
| Sertraline | 100 | Lithium | 100 |
| Vortioxetine | 100 | Bupropion | 80 |
| Desvenlafaxine | 100 | Mirtazapine | 60 |
| Escitalopram | 80 | Mianserin | 60 |
| Duloxetine | 80 | Modafinil | 80 |
| Citalopram | 80 | Armodafinil | 80 |
| Paroxetine | 60 | Olanzapine | 60 |
| Vilazodone | 60 | Triiodothyronine | 80 |
| Agomelatine | 60 | Ketamine | 80 |
| Nortriptyline | 40 | Methylphenidate | 60 |
| Clomipramine | 40 | Lisdexamfetamine | 60 |
| Desipramine | 40 | Ziprasidone | 60 |
| Imipramine | 40 | Lurasidone | 60 |
| Trimipramine | 40 | Cariprazine | 80 |
| Doxepin | 40 | Esketamine | 80 |
| Amitriptyline | 40 | | |
| Mirtazapine | 40 | | |
| Tranylcypromine | 40 | | |
| Trazodone | 20 | | |
| Quetiapine | 20 | | |
| Mianserin | | | 20 |

Getting a score of 100 for both venlafaxine and aripiprazole.

BP - Blood Parameters: The patient does not present values compatible with the parameters included in the algorithm (decreased hematocrit, decreased hemoglobin, anemia, leukopenia, elevated hematocrit, liver dysfunction, hepatic insufficiency, renal dysfunction, hypothyroidism, hyperthyroidism, renal insufficiency, risk of bleeding, risk of venous thromboembolism) so the score of 100 is assigned to both venlafaxine and aripiprazole.

PK - Pharmacokinetics: Venlafaxine: The main metabolization pathway is through the enzyme CYP2D6 which, according to the patient's genotype, is functioning normally. This result means that the patient has two copies of normal function alleles. Based on the genotype result, there is no reason to selectively adjust the dose and a score of 100 is assigned. Aripiprazole: The CYP3A4 and CYP2D6 metabolization pathways are normal according to its genotype. Based on the genotype result, there is no reason to selectively adjust the dose and a score of 100 is assigned.

PD - Pharmacodynamics: Venlafaxine has 40 PharmGKB annotations. 11 annotations suggest greater efficacy, 7 intermediate efficacy, and 15 suggest decreased efficacy. 3 annotations suggest a greater risk of toxicity, 1 intermediate risk, and 3 low risk. This means that in the toxicity category, it obtained 35 points (3x0+1x5+3x10) out of 70 available points (3x10+1x10+3x10), which is 50%, and a score of 25 is assigned for toxicity. Similarly, in efficacy, it obtained 145 points (11x10+7x5+15x0) out of 320 points available (11x10+7x10+15x10), 45%, so a score of 12 is assigned. Then both scores are summed to obtain a total of 37 for venlafaxine.

Aripiprazole has 6 annotations. 2 annotations suggest greater efficacy. 1 annotation suggests a lower risk of toxicity, 1 intermediate risk, and 2 high risk. This means that in the toxicity category, it obtained 15 points (2x0+1x5+1x10) out of 40 available points (2x10+1x5+1x10), which is 37.5%, and a score of 12 is assigned for toxicity. Similarly, it obtained 20 points (2x10) out of 20 points available, 100%, so a score of 50 is assigned. Then both scores are summed to obtain a total of 62 for aripiprazole.

7. The final score for each drug is calculated by multiplying the scores of each attribute by its weight and summing them, resulting in the following table:

| **Drug** | **Type** | **RE score** | **SE score** | **PS score** | **PK score** | **PD score** | **Total Score** |
|---|---|---|---|---|---|---|---|
| | | *weight 0.30* | *weight 0.20* | *weight 0.18* | *weight 0.27* | *weight 0.05* | |
| Agomelatine | Antidepressant | 90 | 60 | 100 | 80 | 49 | 81.05 |
| Bupropion | Antidepressant | 90 | 100 | 100 | 100 | 62 | 95.1 |
| Bupropion | Adjunctive | 70 | 60 | 100 | 100 | 62 | 81.1 |
| Citalopram | Antidepressant | 90 | 80 | 100 | 50 | 24 | 75.7 |
| Escitalopram | Antidepressant | 100 | 80 | 100 | 50 | 49 | 79.95 |
| Fluoxetine | Antidepressant | 90 | 100 | 100 | 100 | 62 | 95.1 |
| Fluvoxamine | Antidepressant | 80 | 100 | 100 | 100 | 24 | 90.2 |
| Paroxetine | Antidepressant | 90 | 60 | 100 | 100 | 49 | 86.45 |
| Sertraline | Antidepressant | 95 | 100 | 100 | 80 | 37 | 89.95 |
| Desvenlafaxine | Antidepressant | 90 | 100 | 100 | 100 | 62 | 95.1 |
| Duloxetine | Antidepressant | 80 | 80 | 100 | 90 | 49 | 84.75 |
| Venlafaxine | Antidepressant | 95 | 100 | 100 | 100 | 37 | 95.35 |
| Mirtazapine | Antidepressant | 95 | 40 | 100 | 90 | 49 | 81.25 |
| Mianserin | Antidepressant | 80 | 20 | 100 | 100 | 49 | 75.45 |
| Mirtazapine | Adjunctive | 70 | 80 | 100 | 40 | 49 | 68.25 |
| Mianserin | Adjunctive | 70 | 80 | 100 | 40 | 49 | 68.25 |
| Vortioxetine | Antidepressant | 95 | 100 | 100 | 100 | 10 | 94 |
| Amitriptyline | Antidepressant | 70 | 40 | 100 | 50 | 49 | 62.95 |
| Nortriptyline | Antidepressant | 60 | 40 | 100 | 100 | 49 | 73.45 |
| Clomipramine | Antidepressant | 60 | 40 | 100 | 50 | 50 | 60 |
| Desipramine | Antidepressant | 60 | 40 | 100 | 50 | 49 | 59.95 |
| Doxepin | Antidepressant | 60 | 40 | 100 | 50 | 62 | 60.6 |
| Imipramine | Antidepressant | 60 | 40 | 100 | 50 | 49 | 59.95 |
| Trimipramine | Antidepressant | 60 | 40 | 100 | 50 | 49 | 59.95 |
| Quetiapine | Antidepressant | 60 | 20 | 100 | 100 | 75 | 70.75 |
| Quetiapine | Adjunctive | 100 | 60 | 100 | 100 | 75 | 90.75 |
| Aripiprazole | Adjunctive | 100 | 100 | 100 | 100 | 62 | 98.1 |
| Ziprasidone | Adjunctive | 40 | 60 | 100 | 100 | 17 | 69.85 |
| Lurasidone | Adjunctive | 40 | 60 | 100 | 100 | 10 | 69.5 |
| Cariprazine | Adjunctive | 40 | 80 | 100 | 100 | 10 | 73.5 |
| Trazodone | Antidepressant | 60 | 20 | 100 | 100 | 49 | 69.45 |
| Tranylcypromine | Antidepressant | 40 | 40 | 100 | 100 | 49 | 67.45 |
| Vilazodone | Antidepressant | 60 | 60 | 100 | 100 | 10 | 75.5 |
| Risperidone | Adjunctive | 100 | 100 | 100 | 100 | 25 | 69.25 |
| Brexpiprazole | Adjunctive | 70 | 100 | 100 | 100 | 10 | 86.5 |
| Modafinil | Adjunctive | 70 | 80 | 100 | 100 | 10 | 82.5 |
| Methylphenidate | Adjunctive | 40 | 60 | 100 | 100 | 30 | 70.5 |
| Lisdexamfetamine | Adjunctive | 40 | 60 | 100 | 100 | 10 | 69.5 |
| Armodafinil | Adjunctive | 40 | 80 | 100 | 100 | 10 | 73.5 |
| Olanzapine | Adjunctive | 70 | 60 | 100 | 90 | 49 | 77.75 |
| Triiodothyronine | Adjunctive | 70 | 80 | 100 | 100 | 10 | 82.5 |
| Ketamine | Adjunctive | 40 | 80 | 100 | 100 | 10 | 73.5 |
| Lithium | Adjunctive | 70 | 100 | 100 | 100 | 17 | 86.85 |
| Esketamine | Adjunctive | 40 | 80 | 100 | 100 | 10 | 73.5 |

### Example 2

Female, 26 years old. Initiates pharmacological treatment for mild unipolar depression. Blood parameters with values compatible with anemia. She does not report any underlying conditions. She presents sleep disturbances (insomnia) and eating disorders, with weight gain secondary to the depressive episode.

It is observed that the CYP2C19 enzyme exhibits intermediate metabolism. For some specific drugs, whose main metabolism pathway is CYP2C19, having intermediate metabolism impacts clinical response. In this case, there could be a slowed metabolism of the involved drugs with an increased possibility of side effects. Based on the results considering not only genetic variables but also different biomarkers, the following drugs are found as the first-line treatment options for antidepressants: venlafaxine, agomelatine, fluoxetine, vortioxetine, and fluvoxamine are considered as first-line antidepressants. Among the adjunctive agents, the following are considered as first-line options: aripiprazole, quetiapine, risperidone, lithium, and brexpiprazole.

When evaluating the use of these drugs from the perspective of sleep and eating disorders, based on the available information, priority was given to those with minimal effect on weight gain and moderate to high somnolence. The algorithm analysis considered body mass index, weight changes secondary to the depressive episode, and sleep disturbances.

While awaiting the results of the application of this method, pharmacological treatment with sertraline was initiated. The patient experienced side effects at low doses, so the treatment had to be discontinued. These findings are consistent with the genetic variations found (alteration of the CYP2C19 enzyme). Subsequently, and with the results of the application of this method, pharmacological treatment with vortioxetine was implemented, achieving remission at 12 weeks. (67% reduction in SIGH-D-17 scale).

### Detail:

1. Compilation of clinical parameters (Data reported by the medical professional and by the patient).

For the origin of obtaining the value of each parameter, see the section "Biomarker Group 1 - Phenotypes - Clinical Parameters" above.

Patient ID: 6. Calculation: the sequential number assigned by the system.

Gender: Female. Calculation: The patient selects from the dropdown menu.

Date of birth: September 30, 1996. Calculation: The patient selects from the calendar menu.

Age: 25. Calculation: calculated by the system.

Pregnancy: No. Calculation: automatic based on gender.

Body Mass Index (BMI): Normal weight. Calculation: assigned by the system as height/weight^2 falls between 18.5 and 24.9.

Age range: Adult, Sleep disorder: Insomnia, Weight change: Increase, Smoker: No, Bipolar spectrum: No, Depression severity: Moderately severe, Underlying condition: None, Current medications: Sertraline.

2. Compilation of blood parameters (Data obtained from the patient's blood sample).

For the origin of obtaining the value of each parameter, see the section "Biomarker Group 2 - Phenotypes - Blood Parameters" above.

It is obtained from the blood analysis.

White blood cell count: Normal, neutrophils: Normal, eosinophils: Normal, basophils: Normal, lymphocytes: Normal, monocytes: Normal, blood glucose: Normal, urea: Normal, creatinine: Normal, total cholesterol: Normal, AST: Normal, ALT: Normal, bilirubin: Normal, renal dysfunction: No, renal failure: No, hepatic dysfunction: No, hepatic failure: No, Anemia: No, Hyperthyroidism: No, Hypothyroidism: No, Hyponatremia: No.

Leukopenia: No, hemoglobin: Normal, hematocrit: Normal, alkaline phosphatase: Normal, proteins: Normal, albumin: Normal, TSH: Elevated, T3: Normal, T4L: Normal, triglycerides: Normal, HDL cholesterol: Normal, LDL cholesterol: Normal, platelet count: Normal, prothrombin concentration: Normal, INR: Normal, partial thromboplastin time: Normal, sodium level: Normal, potassium level: Normal.

3. Compilation of genetic parameters (Data obtained from the patient's DNA sample).

For the origin of obtaining the value of each parameter, see the section "Biomarker Group 3 - Genotypes - Blood Parameters" above.

Among the genetic variations, the polymorphisms rs12769205-AG, rs3758581-GG, and rs4244285-AG are found in the CYP2C19 gene, and it is an intermediate metabolizer CYP2C19 *1/2. Also, the genetic variations rs887829-CT, UGT1A1 *1/*80 decreased and rs2011425-GT, UGT1A4 *1A/3B increased. On the other hand, normal metabolism is found in CYP1A21A/*1A, CYP3A4 *1/*1, CYP2D6 *1/*1, and CYP2C9 *1/*1. Metabolism via CYP2B6 *4/*9 is intermediate (rs2279343-AG, rs3745274-GT).

In addition to the genetic variations of the cytochrome P450 and UGT genes, like in the previous case, the panel used for the patient contains 157 genetic variations that generate the annotations available in PharmGKB for drugs. The annotations report the association between a genetic variant and a drug phenotype from a single publication.

4. First step of the Multicriteria Decision Analysis (MCDA) methodology. Determination of the attributes used in MCDA. Considering that the person is female and has no underlying pathologies, according to the considerations for determining the attributes used in MCDA described previously (see Methodology Step # 1), the attributes for analysis are Age Range (AR), Blood Parameters (BP), Pharmacokinetics (PK), Pharmacodynamics (PD), and Side Effects (SE).

5. Second step of the MCDA methodology. Determination of the weights for each attribute. Considering again that the person is male and has no underlying pathologies, according to the table in Methodology Step # 2, we obtain the following weights for the attributes: AR: 0.30, PK: 0.27, BP: 0.18, PD: 0.05, and SE: 0.20.

6. Third step of the MCDA methodology. Scoring criteria for each attribute. Considering the criteria established for assigning scores to each attribute (see Methodology Step # 3), they are assigned to each drug. For example, the scores for fluoxetine and risperidone are assigned as follows:

AR - Age range: Fluoxetine is a first-line antidepressant suggested for the treatment of unipolar depression by CANMAT clinical guidelines and is the treatment of choice KMAP, consequently, it is assigned a score of 90.

Risperidone is a second-generation antipsychotic suggested as a first-line adjunctive agent for the treatment of unipolar depression by CANMAT clinical guidelines, and it is assigned a score of 100.

SE - Side effects: For algorithm analysis, body mass index, weight changes secondary to depressive episodes, and sleep disturbances are considered. The patient has Normal weight, with weight gain secondary to the depressive episode and sleep disorder (insomnia). When evaluating the use of fluoxetine from the perspective of sleep and eating disturbances, according to available information, it is considered a drug with minimal effect on weight gain and moderate drowsiness. The ranking number for drug evaluation is obtained from the same tables as the previous case. When evaluating the use of risperidone from the perspective of sleep and eating disturbances, according to available information, it is considered a drug with an effect of weight gain and moderate drowsiness. The rankings obtained are number 15 for antidepressants and 15 for adjunctive agents.

| Ranking Number 15 | | Ranking Number 15 | |
|---|---|---|---|
| Antidepressant | Score | Adjunctive agent | Score |
| Fluoxetine | 100 | Aripiprazole | 80 |
| Venlafaxine | 100 | Quetiapine | 80 |
| Fluvoxamine | 100 | Risperidone | 80 |
| Sertraline | 100 | Brexpiprazole | 80 |
| Vortioxetine | 100 | Lithium | 80 |
| Desvenlafaxine | 100 | Bupropion | 60 |
| Agomelatine | 100 | Mirtazapine | 60 |
| Escitalopram | 80 | Mianserin | 60 |
| Duloxetine | 80 | Modafinil | 60 |
| Citalopram | 80 | Armodafinil | 60 |
| Trazodone | 60 | Olanzapine | 60 |
| Mirtazapine | 60 | Triiodothyronine | 60 |
| Paroxetine | 60 | Ketamine | 60 |
| Vilazodone | 60 | Methylphenidate | 40 |
| Bupropion | 40 | Lisdexamfetamine | 40 |
| Nortriptyline | 40 | Ziprasidone | 40 |
| Clomipramine | 40 | Lurasidone | 80 |
| Imipramine | 40 | Cariprazine | 60 |
| Trimipramine | 40 | Esketamine | 60 |
| Doxepin | 40 | | |
| Amitriptyline | 40 | | |
| Desipramine | 20 | | |
| Quetiapine | 40 | | |
| Mianserin | 40 | | |
| Tranylcypromine | 40 | | |

Obtaining a score of 100 for fluoxetine and 80 for risperidone.

BP - Blood Parameters: The patient does not present values compatible with the parameters included at the time in the algorithm (decreased hematocrit, decreased hemoglobin, anemia, leukopenia, elevated hematocrit, hepatic dysfunction, hepatic insufficiency, renal dysfunction, hypothyroidism, hyperthyroidism, renal insufficiency, risk of bleeding, risk of venous thromboembolism), so a score of 100 is assigned to both fluoxetine and risperidone.

PK - Pharmacokinetics: Fluoxetine and risperidone. The main metabolic pathway is through the CYP2D6 enzyme, which functions normally. This result indicates that the patient has two copies of normal function alleles. According to the genotype result, there is no reason to selectively adjust the dose, so a score of 100 is assigned to both.

PD - Pharmacodynamics: Fluoxetine has 39 annotations. 14 annotations suggest higher efficacy, 8 intermediate efficacy, and 11 annotations suggest low efficacy. 2 annotations suggest a low risk of toxicity, 1 an intermediate risk, and 3 annotations a low risk. This means that in the efficacy category, it obtained 180 points (14x10+8x5+11x0) out of the 330 available (14x10+8x10+11x10), which is 54%, and it is assigned a score of 37 for efficacy. Similarly, in toxicity, it obtained 25 points (2x10+1x5+3x0) out of 60 points (2x10+1x10+3x10), which is 41%, so it is assigned a score of 12. Then both scores are summed to obtain a total of 49 for fluoxetine.

Risperidone has 14 annotations. 4 annotations suggest lower efficacy. 5 annotations suggest a higher risk of toxicity, 1 an intermediate risk, and 4 a decreased risk. This means that in the toxicity category, it obtained 45 points (5x0+1x5+4x10) out of 100 available (5x10+1x10+4x10), which is 45%, so it is assigned a score of 12 for toxicity. Similarly, it obtained 0 points in efficacy (4x0) out of the 40 points available, which is 0%, so it is assigned a score of 0. Then both scores are summed to obtain a total of 12 for risperidone.

7. The final score for each drug is calculated by multiplying the scores of each attribute by its weight and summing them, resulting in the following table:

| **Drug** | **Type** | **RE score** *weight 0.30* | **SE score** *weight 0.20* | **BP score** *weight 0.18* | **PK score** *weight 0.27* | **PD score** *weight 0.05* | Total Score |
|---|---|---|---|---|---|---|---|
| Agomelatine | Agomelatine | 90 | 100 | 100 | 100 | 50 | 94.5 |
| Bupropion | Bupropion | 90 | 40 | 100 | 80 | 74 | 78.3 |
| Citalopram | Citalopram | 90 | 80 | 100 | 80 | 49 | 85.05 |
| Desvenlafaxine | Desvenlafaxin e | 90 | 100 | 100 | 80 | 62 | 89.7 |
| Duloxetine | Duloxetine | 80 | 80 | 100 | 100 | 37 | 86.85 |
| Escitalopram | Escitalopram | 100 | 80 | 100 | 80 | 37 | 87.45 |
| Fluoxetine | Fluoxetine | 90 | 100 | 100 | 100 | 49 | 94.45 |
| Fluvoxamine | Fluvoxamine | 80 | 100 | 100 | 100 | 37 | 90.85 |
| Mirtazapine | Mirtazapine | 95 | 60 | 100 | 100 | 50 | 88 |
| Paroxetine | Paroxetine | 90 | 60 | 100 | 100 | 50 | 86.5 |
| Sertraline | Sertraline | 95 | 100 | 100 | 80 | 50 | 90.6 |
| Venlafaxine | Venlafaxine | 95 | 100 | 100 | 100 | 49 | 95.95 |
| Vortioxetine | Vortioxetine | 95 | 100 | 100 | 100 | 10 | 94 |
| Amitriptyline | Amitriptyline | 70 | 40 | 100 | 80 | 62 | 71.7 |
| Nortriptyline | Nortriptyline | 60 | 40 | 100 | 50 | 24 | 58.7 |
| Clomipramine | Clomipramine | 60 | 40 | 100 | 80 | 62 | 68.7 |
| Desipramine | Desipramine | 60 | 20 | 100 | 50 | 37 | 55.35 |
| Doxepin | Doxepin | 60 | 40 | 100 | 80 | 62 | 68.7 |
| Imipramine | Imipramine | 60 | 40 | 100 | 50 | 37 | 59.35 |
| Trimipramine | Trimipramine | 60 | 40 | 100 | 50 | 37 | 59.35 |
| Trazodone | Trazodone | 60 | 60 | 100 | 100 | 62 | 78.1 |
| Vilazodone | Vilazodone | 60 | 60 | 100 | 100 | 10 | 75.5 |
| Tranylcypromine | Tranylcypromi ne | 40 | 40 | 100 | 100 | 37 | 66.85 |
| Mianserin | Mianserin | 80 | 40 | 100 | 90 | 37 | 76.15 |
| Quetiapine | Quetiapine | 60 | 40 | 100 | 100 | 87 | 75.35 |
| Quetiapine | Quetiapine | 100 | 80 | 100 | 100 | 87 | 95.35 |
| Aripiprazole | Aripiprazole | 100 | 80 | 100 | 100 | 87 | 95.35 |
| Risperidone | Risperidone | 100 | 80 | 100 | 100 | 12 | 91.6 |
| Brexpiprazole | Brexpiprazole | 70 | 80 | 100 | 100 | 10 | 82.5 |
| Bupropion | Bupropion | 70 | 60 | 100 | 80 | 74 | 76.3 |
| Mirtazapine | Mirtazapine | 70 | 60 | 100 | 100 | 50 | 80.5 |
| Mianserin | Mianserin | 70 | 60 | 100 | 100 | 37 | 79.85 |
| Modafinil | Modafinil | 70 | 60 | 100 | 100 | 10 | 78.5 |
| Olanzapine | Olanzapine | 70 | 60 | 100 | 100 | 62 | 81.1 |
| Triiodothyronine | Triiodothyronin | 70 | 60 | 100 | 100 | 10 | 78.5 |
| Methylphenidate | Methylphenida te | 40 | 40 | 100 | 100 | 30 | 66.5 |
| Lisdexamfetamine | Lisdexamfeta mine | 40 | 40 | 100 | 100 | 10 | 65.5 |
| Ziprasidone | Ziprasidone | 40 | 40 | 100 | 100 | 42 | 67.1 |
| Ketamine | Ketamine | 40 | 60 | 100 | 100 | 10 | 69.5 |
| Lithium | Lithium | 70 | 80 | 100 | 100 | 49 | 84.45 |
| Armodafinil | Armodafinil | 40 | 60 | 100 | 100 | 10 | 69.5 |
| Esketamine | Esketamine | 40 | 60 | 100 | 90 | 10 | 66.8 |
| Lurasidone | Lurasidone | 40 | 80 | 100 | 100 | 10 | 73.5 |
| Cariprazine | Cariprazine | 40 | 60 | 100 | 100 | 10 | 69.5 |

### Example 3

Female person, 42 years old. Diagnosed with treatment-resistant unipolar depression, SIGH-D-17: 21 (Moderate), PHQ9: 22 (Severe), comorbid with attention deficit disorder.

She has Type I obesity without weight change secondary to depression, and sleep disturbance (insomnia). Blood parameters show values consistent with anemia. Anemia could negatively affect the patient's symptomatology, suggesting investigating causes for correction.

As for pharmacological history, she reports no response to escitalopram, desvenlafaxine, cariprazine, clomipramine, vortioxetine, and vilazodone. The current pharmacological regimen consists of venlafaxine, lithium, clonazepam, and zolpidem. The person also uses psilocybin.

No blood parameters have been observed to interfere with drug response.

Regarding genetic data, it has been observed that the CYP2C19 metabolism enzyme in this patient metabolizes drugs slowly, and the CYP2B6 enzyme is an intermediate metabolizer. This results in the accumulation of drugs that use this metabolism pathway, leading to a higher incidence of side effects and/or toxicity.

Algorithm result: The following drugs are recommended: venlafaxine, fluoxetine, desvenlafaxine, vortioxetine, and fluvoxamine. The use of drugs primarily metabolized by other pathways is suggested, for example, venlafaxine, whose main metabolism pathway is CYP2D6 and adjusts to the clinical profile (degree of depression, sleep, and eating disturbances, patient's comorbidities, and blood parameters). Fluoxetine or desvenlafaxine could be considered in the second instance.

Among adjunctive agents, aripiprazole, risperidone, and quetiapine are considered mainly for their metabolism pathway (not involving the enzyme (CYP2C19) and pharmacodynamics. Brexpiprazole could also be considered because it is approved and recommended by guidelines for treatment-resistant depression. The lower scores for this drug result from it being relatively new in the market, with fewer studies on its pharmacodynamics.

Due to potential interactions (zolpidem, clonazepam, and quetiapine) and the suggestions from the method's results for brexpiprazole, quetiapine is gradually replaced by brexpiprazole. The response was observed at 8 weeks of treatment. (53% reduction in SIGH-D-17 scale).

### Detail:

1. Compilation of clinical parameters (Data reported by the medical professional and by the patient).

For the origin of obtaining the value of each parameter, see the section "Biomarker Group 1 - Phenotypes - Clinical Parameters" above).

Patient ID: 8. Gender: Female. Date of birth: March 30, 1980. Age: 42. Pregnancy: No. Body Mass Index (BMI): Obesity type I. Calculation: assigned by the system for the height/weight^2 being between 30 and 34.9.

Age range: adult, sleep disorder: Insomnia, Weight Change: No, Smoker: Yes, Cognitive Functions: Yes, Bipolar Spectrum: No, Depression Severity: Severe, Underlying Condition: Attention Deficit Disorder, Current Medications: Quetiapine, venlafaxine, lithium, clonazepam, zolpidem, psilocybin.

2. Compilation of blood parameters (Data obtained from the patient's blood sample).

For the origin of obtaining the value of each parameter, see the section "Biomarker Group 2 - Phenotypes - Blood Parameters" above).

It is obtained from the blood analysis.

White blood cell count: Normal, neutrophils: Normal, eosinophils: Normal, basophils: Normal, lymphocytes: Normal, monocytes: Normal, blood glucose: Normal, urea: Normal, creatinine: Normal, total cholesterol: Elevated, AST: Normal, ALT: Normal, ALT: Normal, bilirubin: Normal, renal dysfunction: No, renal insufficiency: No, hepatic dysfunction: No, hepatic insufficiency: No, Anemia: No, Hyperthyroidism: No, Hypothyroidism: No, Hyponatremia: No.

Leukopenia: No, hemoglobin: Decreased, hematocrit: Decreased, alkaline phosphatase: Normal, proteins: Normal, albumin: Normal, TSH: Elevated, T3: Normal, T4L: Normal, triglycerides: Elevated, HDL cholesterol: Normal, LDL cholesterol: Normal, platelet count: Normal, prothrombin concentration: Normal, INR: Normal, partial thromboplastin time: Normal, sodium level: Normal, potassium level: Normal.

3. Compilation of genetic parameters (Data obtained from the patient's DNA sample).

For the origin of obtaining the value of each parameter, see the section "Biomarker Group 3 - Genotypes - Blood Parameters" above).

Among the genetic variations, there is CYP2B6 *4/*9, an intermediate metabolizer through this pathway. The CYP1A2 *1A/*1F, CYP3A4 *1/*1, CYP2C9 *1/*1, UGT1A1 *1A/*1A, and UGT1A4 *1/*1 are normal metabolizers. CYP2C19 presents the polymorphisms rs28399504-GG and rs3758581-GG and is a slow metabolizer CYP2C19 *4/4.

In addition to the genetic variations of the cytochrome P450 and UGT genes, like the previous case, the panel used for the patient contains 157 genetic variations that generate the annotations available in PharmGKB for the drugs. The annotations report the association between a genetic variant and a drug phenotype from a single publication.

4. First step of the Multicriteria Decision Analysis (MCDA) methodology. Determination of the attributes used in MCDA. Considering that the individual is a female, adult, not pregnant, and has underlying conditions, according to the considerations for determining the attributes used in MCDA described earlier (see Methodology Step #1), the attributes for analysis are Underlying Conditions (UC), Age Range (AR), Blood Parameters (BP), Pharmacokinetics (PK), Pharmacodynamics (PD), and Side Effects (SE).

5. Second step of the MCDA methodology. Determination of the weights for each attribute. Considering again that the individual is male and has underlying conditions, according to the table in Methodology Step #2, we obtain the following weights for the attributes: UC: 0.25, AR: 0.17, PK: 0.22, BP: 0.15, PD: 0.05, and SE: 0.16.

6. Third step of the MCDA methodology. Scoring criteria for each attribute. Considering the established criteria for assigning scores to each attribute (see Methodology Step #3), they are assigned for each drug. For example, the scores for vortioxetine and brexpiprazole are assigned as follows:

| Ranking Number 17 | | Ranking Number 4 | |
|---|---|---|---|
| Antidepressants | Score | Adjunctive agent | Score |
| Bupropion | 100 | Aripiprazole | 100 |
| Fluoxetine | 100 | Quetiapine | 40 |
| Venlafaxine | 100 | Risperidone | 100 |
| Fluvoxamine | 100 | Lithium | 100 |
| Sertraline | 100 | Mirtazapine | 100 |
| Vortioxetine | 100 | Mianserin | 100 |
| Desvenlafaxine | 100 | Olanzapine | 60 |
| Agomelatine | 60 | Brexpiprazole | 80 |
| Escitalopram | 60 | Ketamine | 80 |
| Duloxetine | 60 | Ziprasidone | 80 |
| Citalopram | 60 | Bupropion | 60 |
| Paroxetine | 60 | Modafinil | 60 |
| Vilazodone | 60 | Armodafinil | 60 |
| Mirtazapine | 40 | Triiodothyronine | 60 |
| Nortriptyline | 20 | Methylphenidate | 60 |
| Clomipramine | 20 | Lisdexamfetamine | 60 |
| Desipramine | 20 | Lurasidone | 80 |
| Imipramine | 20 | Cariprazine | 60 |
| Trimipramine | 20 | Esketamine | 60 |
| Doxepin | 20 | | |
| Amitriptyline | 20 | | |
| Trazodone | 20 | | |
| Quetiapine | 10 | | |
| Mianserin | 10 | | |
| Tranylcypromine | 40 | | |

Obtaining a score of 100 for vortioxetine and 80 for brexpiprazole from these.

BP - Blood Parameters: The patient does not present values compatible with the parameters included at the time in the algorithm, so both vortioxetine and brexpiprazole are assigned a score of 100.

PK - Pharmacokinetics: Vortioxetine and brexpiprazole are metabolized primarily by CYP3A4 and CYP2D6, which are normal according to genotype data. Additionally, the CYP2C9 enzyme is involved in the metabolism of vortioxetine. This result indicates that the patient has two copies of normal function alleles. Based on the genotype result, there is no reason to selectively adjust the dose, so a score of 100 is assigned.

PD - Pharmacodynamics: Currently, there are no studies associating vortioxetine or brexpiprazole with genomic alterations linked to pharmacodynamics. Since there are no points available, according to the allocation criteria explained above, vortioxetine and brexpiprazole are attributed a score of 5 for both efficacy and toxicity. Then, both scores are summed to obtain a total of 10 for vortioxetine and 10 for brexpiprazole.

The final score for each drug is calculated by multiplying the scores of each attribute by its weight and summing them, resulting in the following table:

| **Drug** | **Type** | **AR score** *weight* 0.17 | **PB score** *weight 0.25* | **SE score** *weight 0.16* | **BP score** *weight 0.15* | **PK score** *weight 0.22* | **PD score** *weight 0.05* | **Score** |
|---|---|---|---|---|---|---|---|---|
| Agomelatine | Antidepressant | 90 | 100 | 60 | 100 | 80 | 37 | 84.35 |
| Bupropion | Antidepressant | 90 | 100 | 100 | 100 | 80 | 49 | 91.35 |
| Citalopram | Antidepressant | 90 | 100 | 60 | 100 | 50 | 62 | 79 |
| Desvenlafaxine | Antidepressant | 90 | 100 | 100 | 100 | 100 | 49 | 95.75 |
| Duloxetine | Antidepressant | 80 | 100 | 60 | 100 | 100 | 24 | 86.4 |
| Escitalopram | Antidepressant | 100 | 100 | 60 | 100 | 50 | 37 | 79.45 |
| Fluoxetine | Antidepressant | 90 | 100 | 100 | 100 | 100 | 50 | 95.8 |
| Fluvoxamine | Antidepressant | 80 | 100 | 100 | 100 | 100 | 49 | 94.05 |
| Mirtazapine | Antidepressant | 95 | 100 | 40 | 100 | 100 | 24 | 85.75 |
| Paroxetine | Antidepressant | 90 | 100 | 60 | 100 | 100 | 24 | 88.1 |
| Sertraline | Antidepressant | 95 | 100 | 100 | 100 | 50 | 24 | 84.35 |
| Venlafaxine | Antidepressant | 95 | 100 | 100 | 100 | 100 | 49 | 96.6 |
| Vortioxetine | Antidepressant | 95 | 100 | 100 | 100 | 100 | 10 | 94.65 |
| Amitriptyline | Antidepressant | 70 | 100 | 20 | 100 | 50 | 49 | 68.55 |
| Nortriptyline | Antidepressant | 60 | 100 | 20 | 100 | 100 | 37 | 77.25 |
| Clomipramine | Antidepressant | 60 | 100 | 20 | 100 | 50 | 49 | 66.85 |
| Desipramine | Antidepressant | 60 | 100 | 20 | 100 | 50 | 49 | 66.85 |
| Doxepin | Antidepressant | 60 | 100 | 20 | 100 | 50 | 49 | 66.85 |
| Imipramine | Antidepressant | 60 | 100 | 20 | 100 | 60 | 24 | 67.8 |
| Trimipramine | Antidepressant | 60 | 100 | 20 | 100 | 60 | 24 | 67.8 |
| Trazodone | Antidepressant | 60 | 100 | 20 | 100 | 100 | 50 | 77.9 |
| Vilazodone | Antidepressant | 60 | 100 | 60 | 100 | 100 | 10 | 82.3 |
| Tranylcypromine | Antidepressant | 40 | 100 | 10 | 100 | 100 | 24 | 71.6 |
| Mianserin | Antidepressant | 80 | 100 | 40 | 100 | 100 | 100 | 71.3 |
| Quetiapine | Antidepressant | 60 | 100 | 10 | 100 | 100 | 62 | 68.4 |
| Quetiapine | Adjunctive | 100 | 100 | 60 | 100 | 100 | 62 | 88.5 |
| Aripiprazole | Adjunctive | 100 | 100 | 100 | 100 | 100 | 37 | 96.85 |
| Risperidone | Adjunctive | 100 | 100 | 100 | 100 | 100 | 24 | 96.2 |
| Brexpiprazole | Adjunctive | 70 | 100 | 80 | 100 | 100 | 10 | 87.2 |
| Bupropion | Adjunctive | 70 | 100 | 60 | 100 | 80 | 49 | 81.55 |
| Mirtazapine | Adjunctive | 70 | 100 | 100 | 100 | 100 | 24 | 91.1 |
| Mianserin | Adjunctive | 70 | 100 | 100 | 100 | 100 | 24 | 91.1 |
| Modafinil | Adjunctive | 70 | 100 | 60 | 100 | 100 | 10 | 84 |
| Olanzapine | Adjunctive | 70 | 100 | 60 | 100 | 100 | 24 | 84.7 |
| Triiodothyronine | Adjunctive | 70 | 100 | 60 | 100 | 100 | 10 | 84 |
| Methylphenidate | Adjunctive | 40 | 100 | 60 | 100 | 100 | 55 | 81.15 |
| Lisdexamfetamine | Adjunctive | 40 | 100 | 60 | 100 | 100 | 10 | 78.9 |
| Ziprasidone | Adjunctive | 40 | 100 | 80 | 100 | 100 | 17 | 82.45 |
| Ketamine | Adjunctive | 40 | 100 | 80 | 100 | 100 | 10 | 82.1 |
| Lithium | Adjunctive | 70 | 100 | 100 | 100 | 100 | 37 | 91.75 |
| Armodafinil | Adjunctive | 40 | 100 | 60 | 100 | 100 | 10 | 78.9 |
| Esketamine | Adjunctive | 40 | 100 | 60 | 100 | 90 | 10 | 76.7 |
| Lurasidone | Adjunctive | 40 | 100 | 80 | 100 | 100 | 10 | 82.1 |
| Cariprazine | Adjunctive | 40 | 100 | 60 | 100 | 100 | 10 | 78.9 |

### Example 4

Female, 58 years old. Currently experiencing a depressive episode, with a SIGH-D-17 score of 37 (Moderate) and a PHQ-9 score of 20 (Severe). Clinical parameters indicate sleep disturbance (hypersomnia) and smoking. Comorbidities include lactose intolerance, hypothyroidism, hypertension, and hypercholesterolemia.

She has normal weight without weight change secondary to unipolar depression. Blood parameters show values consistent with hypothyroidism (elevated TSH and decreased T4L), as well as values indicative of anemia, mild hypochromia, and moderate anisocytosis. It is important to consider that these altered values may interfere with the mood disorder, exacerbating depressive symptoms.

Previous pharmacological history includes poor response to clonazepam. Current medication regimen includes citalopram, quetiapine, atorvastatin, esomeprazole, clonazepam, zopiclone, ibuprofen, and diclofenac. She reports subtherapeutic response and side effects. Venlafaxine and lamotrigine were previously ineffective.

Considering the pharmacokinetic parameters from a genetic perspective, variations suggesting probable rapid metabolism in the CYP2C19 and CYP1A2 enzymes of the P450 complex were observed, along with decreased activity in UGT1A1 and increased activity in UGT1A4. The lack of response to citalopram could be explained by rapid metabolism by its main metabolic enzyme (CYP2C19), while the lack of response to lamotrigine could be due to UGT1A4 enzyme alteration. It is important to note that tobacco consumption increases the activity of the CYP1A2 enzyme, which in this patient would have increased genetic action. Therefore, it is suggested to avoid drugs metabolized by this pathway.

Considering both pharmacokinetic and pharmacodynamic biomarkers (pharmacodynamic annotations are specified for each particular drug), the top 5 drugs with which she may have a better response are fluoxetine, vortioxetine, mianserin, vilazodone, and bupropion.

Regarding adjunctive agents, those with the highest score were aripiprazole, risperidone, lithium, brexpiprazole, and modafinil.

It is important to note that commercial preparations of quetiapine often contain lactose, so it is not recommended for individuals with lactose intolerance.

After 8 weeks without response, the results of this method's application were reported to the healthcare professional, who replaced citalopram with vortioxetine and discontinued quetiapine.

### Detail:

1. Compilation of clinical parameters (Data reported by the medical professional and by the patient).

For the origin of obtaining the value of each parameter, see the section "Biomarker Group 1 - Phenotypes - Clinical Parameters" above.

Patient ID: 18. Gender: Female. Date of Birth: April 14, 1964. Age: 58. Pregnancy: No. Body Mass Index (BMI): Normal weight. Calculation: assigned by the system for having a height/weight^2 between 18.5 and 24.9.

Age range: adult, sleep disorder: hypersomnia, Weight Change: No, Smoker: Yes, Bipolar Spectrum: No, Depression Severity: Severe, Underlying conditions: Lactose intolerance, hypertension, hypercholesterolemia, Current medications: citalopram, quetiapine, atorvastatin, esomeprazole, clonazepam, zopiclone, ibuprofen, diclofenac, History: Venlafaxine, lamotrigine.

2. Compilation of blood parameters (Data obtained from the patient's blood sample).

For the origin of obtaining the value of each parameter, see section "Biomarker Group 2 - Phenotypes - Blood Parameters" above).

It is obtained from blood analysis.

White blood cell count: Normal, neutrophils: Normal, eosinophils: Normal, basophils: Normal, lymphocytes: Normal, monocytes: Normal, blood glucose: Normal, urea: Normal, creatinine: Normal, total cholesterol: Normal, AST: Normal, AST: Normal, ALT: Normal, bilirubin: Normal, renal dysfunction: No, renal failure: No, hepatic dysfunction: No, hepatic failure: No, Anemia: No, Hyperthyroidism: No, Hypothyroidism: Yes, Hyponatremia: No.

Leukopenia: No, hemoglobin: Normal, hematocrit: Decreased, alkaline phosphatase: Normal, proteins: Normal, albumin: Normal, TSH: Elevated, T3: Normal, Free T4: Decreased, triglycerides: Elevated, HDL cholesterol: Decreased, LDL cholesterol: Borderline, platelet count: Normal, prothrombin concentration: Normal, INR: Normal, partial thromboplastin time: Normal, sodium level: Normal, potassium level: Normal.

3. Compilation of genetic parameters (Data obtained from the patient's DNA sample).

For the origin of obtaining the value of each parameter, see section "Biomarker Group 3 - Genotypes - Blood Parameters" above).

Among the genetic variations, there is CYP2C19 *1/*17 (rs12248560-CT), a rapid metabolizer through this pathway. The gene CYP1A2*1A/*1F also indicates rapid metabolism through this pathway. CYP3A4*1/*1, CYP2C9*1/*1, normal metabolism. UGT1A1*1A/*80 decreased metabolism and UGT1A4*1/*3B rapid metabolizer.

In addition to the genetic variations of the cytochrome P450 and UGT genes, as in the previous case, the panel used for the patient contains 157 genetic variations that generate the annotations available in PharmGKB for drugs. Annotations inform the association between a genetic variant and a drug phenotype from a single publication.

4. First step of the Multicriteria Decision Analysis (MCDA) methodology. Considering that the individual is a female, adult, not pregnant, and has underlying pathologies, according to the considerations for determining the attributes used in MCDA described previously (see Methodology Step #1), the attributes for analysis are Underlying Pathology (UC), Age Range (AR), Blood Parameters (BP), Pharmacokinetics (PK), Pharmacodynamics (PD), and Side Effects (SE).

5. Second step of the MCDA methodology. Determination of the weights for each attribute. Again considering that the individual is male and has underlying pathologies, according to the chart in Methodology Step #2,

| Ranking Number 14 | | Ranking Number 5 | |
|---|---|---|---|
| Antidepressant | Score | Adjuvant | Score |
| Escitalopram | 100 | Aripiprazole | 100 |
| Venlafaxine | 100 | Risperidone | 100 |
| Sertraline | 100 | Brexpiprazole | 100 |
| Bupropion | 100 | Lithium | 100 |
| Desvenlafaxine | 100 | Bupropion | 80 |
| Fluoxetine | 100 | Modafinil | 80 |
| Vortioxetine | 100 | Armodafinil | 80 |
| Duloxetine | 100 | Triiodothyronine | 80 |
| Paroxetine | 100 | Ketamine | 80 |
| Fluvoxamine | 100 | Cariprazine | 80 |
| Agomelatine | 100 | Esketamine | 80 |
| Citalopram | 100 | Quetiapine | 60 |
| Mirtazapine | 60 | Mirtazapine | 60 |
| Vilazodone | 60 | Mianserin | 60 |
| Desipramine | 60 | Olanzapine | 60 |
| Amitriptyline | 40 | Methylphenidate | 60 |
| Nortriptyline | 40 | Lisdexamfetamine | 60 |
| Clomipramine | 40 | Ziprasidone | 60 |
| Imipramine | 40 | Lurasidone | 60 |
| Trimipramine | 40 | | |
| Doxepin | 40 | | |
| Trazodone | 20 | | |
| Quetiapine | 20 | | |
| Mianserin | 20 | | |
| Tranylcypromine | 40 | | |

Obtaining a score of 100 for bupropion and 80 for modafinil.

PS - Blood Parameters: The patient presents values compatible with hypothyroidism. According to the scoring assignment criteria explained above for this attribute, bupropion receives 100 points, as does modafinil.

| Antidepressant | Score Hypothyroidism | Adjunctive agent | Score Hypothyroidism |
|---|---|---|---|
| Agomelatine | 100 | Quetiapine | 100 |
| Bupropion | 100 | Aripiprazole | 100 |
| Citalopram | 100 | Risperidone | 100 |
| Desvenlafaxine | 100 | Brexpiprazole | 100 |
| Duloxetine | 100 | Modafinil | 100 |
| Escitalopram | 100 | Bupropion | 100 |
| Fluoxetine | 100 | Mirtazapine | 100 |
| Fluvoxamine | 100 | Mianserin | 40 |
| Mirtazapine | 100 | Olanzapine | 100 |
| Paroxetine | 100 | Triiodothyronine | 100 |
| Sertraline | 100 | Methylphenidate | 100 |
| Venlafaxine | 100 | Lisdexamfetamine | 100 |
| Vortioxetine | 100 | Ziprasidone | 100 |
| Amitriptyline | 40 | Ketamine | 100 |
| Nortriptyline | 40 | Lithium | 100 |
| Clomipramine | 40 | Armodafinil | 100 |
| Desipramine | 40 | Esketamine | 100 |
| Doxepin | 40 | Lurasidone | 100 |
| Imipramine | 40 | Cariprazine | 100 |
| Trimipramine | 40 | | |
| Quetiapine | 100 | | |
| Trazodone | 100 | | |
| Vilazodone | 100 | | |
| Tranylcypromine | 100 | | |
| Mianserin | 40 | | |

PK - Pharmacokinetics: Bupropion and Modafinil have CYP2B6 and CYP3A4 as their metabolic pathways, which are normal according to their genotype data. According to the genotype result, there is no reason to selectively adjust the dose, and they are assigned a score of 100.

PD - Pharmacodynamics: Bupropion has 86 annotations. 4 annotations suggest a higher risk of toxicity, 12 an intermediate risk, and 8 a low risk. 34 annotations suggest higher efficacy, 2 intermediate efficacy, and 26 reduced efficacy. This means that in the efficacy category, it scored 350 points (34x10+2x5+26x0) out of the available 330 points (34x10+2x10+26x10), that is, 56%, and it is assigned a score of 37 for efficacy. Similarly, in toxicity, it scored 140 points (8x10+12x5+4x0) out of 240 points (8x10+12x10+4x10), 58%, and is assigned a score of 37. Then both scores are added up to obtain a total of 74 for bupropion. For modafinil, there are currently no studies associating genomic alterations with pharmacodynamics. Since there are no points available, according to the allocation criterion explained above, modafinil is assigned a score of 5 for both efficacy and toxicity. Then both scores are added up to obtain a total of 10 for modafinil.

7. The final score for each drug is calculated by multiplying the scores of each attribute by its weight and adding them up, resulting in the following table:

| **Drug** | **Type** | **AR score** *weight 0.17* | **PB score** *weight 0.25* | **SE score** *weight 0.16* | **BP score** *weight 0.15* | **PK score** *weight 0.22* | **PD score** *weight 0.05* | **Score** |
|---|---|---|---|---|---|---|---|---|
| Agomelatine | Antidepressant | 90 | 40 | 100 | 100 | 80 | 74 | 77.6 |
| Bupropion | Antidepressant | 90 | 40 | 100 | 100 | 100 | 74 | 82 |
| Citalopram | Antidepressant | 90 | 40 | 100 | 100 | 50 | 49 | 69.75 |
| Desvenlafaxine | Antidepressant | 90 | 40 | 100 | 100 | 80 | 74 | 77.6 |
| Duloxetine | Antidepressant | 80 | 40 | 100 | 100 | 90 | 74 | 78.1 |
| Escitalopram | Antidepressant | 100 | 40 | 100 | 100 | 50 | 74 | 72.7 |
| Fluoxetine | Antidepressant | 90 | 100 | 100 | 100 | 100 | 74 | 97 |
| Fluvoxamine | Antidepressant | 80 | 40 | 100 | 100 | 100 | 74 | 80.3 |
| Mirtazapine | Antidepressant | 95 | 0 | 60 | 100 | 90 | 74 | 0 |
| Paroxetine | Antidepressant | 90 | 40 | 100 | 100 | 100 | 74 | 82 |
| Sertraline | Antidepressant | 95 | 40 | 100 | 100 | 80 | 74 | 78.45 |
| Venlafaxine | Antidepressant | 95 | 30 | 100 | 100 | 100 | 74 | 80.35 |
| Vortioxetine | Antidepressant | 95 | 100 | 100 | 100 | 100 | 10 | 94.65 |
| Amitriptyline | Antidepressant | 70 | 40 | 40 | 40 | 50 | 74 | 49 |
| Nortriptyline | Antidepressant | 60 | 40 | 40 | 40 | 100 | 74 | 58.3 |
| Clomipramine | Antidepressant | 60 | 40 | 40 | 40 | 50 | 74 | 47.3 |
| Desipramine | Antidepressant | 60 | 40 | 60 | 40 | 100 | 74 | 61.5 |
| Doxepin | Antidepressant | 60 | 40 | 40 | 40 | 50 | 74 | 47.3 |
| Imipramine | Antidepressant | 60 | 40 | 40 | 40 | 100 | 74 | 58.3 |
| Trimipramine | Antidepressant | 60 | 40 | 40 | 40 | 100 | 74 | 58.3 |
| Trazodone | Antidepressant | 60 | 100 | 20 | 100 | 100 | 74 | 79.1 |
| Vilazodone | Antidepressant | 60 | 100 | 60 | 100 | 100 | 10 | 82.3 |
| Tranylcypromine | Antidepressant | 40 | 100 | 40 | 100 | 100 | 74 | 78.9 |
| Mianserin | Antidepressant | 80 | 100 | 20 | 100 | 100 | 74 | 82.5 |
| Quetiapine | Antidepressant | 60 | 0 | 20 | 100 | 100 | 12 | 0 |
| Quetiapine | Adjunctive | 100 | 0 | 60 | 100 | 100 | 12 | 0 |
| Aripiprazole | Adjunctive | 100 | 100 | 100 | 100 | 100 | 25 | 96.25 |
| Risperidone | Adjunctive | 100 | 100 | 100 | 100 | 100 | 24 | 96.2 |
| Brexpiprazole | Adjunctive | 70 | 100 | 100 | 100 | 100 | 10 | 90.4 |
| Bupropion | Adjunctive | 70 | 40 | 80 | 100 | 100 | 74 | 75.4 |
| Mirtazapine | Adjunctive | 70 | 0 | 60 | 100 | 90 | 74 | 0 |
| Mianserin | Adjunctive | 70 | 100 | 60 | 100 | 100 | 74 | 87.2 |
| Modafinil | Adjunctive | 70 | 100 | 80 | 100 | 100 | 10 | 87.2 |
| Olanzapine | Adjunctive | 70 | 100 | 60 | 100 | 90 | 24 | 82.5 |
| Triiodothyronine | Adjunctive | 70 | 100 | 80 | 100 | 100 | 10 | 87.2 |
| Methylphenidate | Adjunctive | 40 | 0 | 60 | 100 | 100 | 55 | 0 |
| Lisdexamfetamine | Adjunctive | 40 | 0 | 60 | 100 | 100 | 10 | 0 |
| Ziprasidone | Adjunctive | 40 | 40 | 60 | 100 | 100 | 17 | 64.25 |
| Ketamine | Adjunctive | 40 | 0 | 80 | 100 | 100 | 10 | 0 |
| Lithium | Adjunctive | 70 | 100 | 100 | 100 | 100 | 49 | 92.35 |
| Armodafinil | Adjunctive | 40 | 100 | 80 | 100 | 100 | 10 | 82.1 |
| Esketamine | Adjunctive | 40 | 40 | 80 | 100 | 100 | 10 | 67.1 |
| Lurasidone | Adjunctive | 40 | 100 | 60 | 100 | 100 | 10 | 78.9 |
| Cariprazine | Adjunctive | 40 | 40 | 80 | 100 | 100 | 10 | 67.1 |

### Adaptation of the Method to Updates in Medical and Technological Fields

The present method of drug selection dynamically adapts to medical and technological advancements, ensuring its relevance and continuous effectiveness in clinical decision-making. This invention provides a systematic and scalable approach to update and improve the process of selecting pharmacological treatments while maintaining its integrity and effectiveness over time.

These updates may be triggered by changes in clinical practices, the introduction of new pharmacological treatments, or the evolution of clinical guidelines and therapeutic protocols. Updates may also be prompted by the need to efficiently handle larger volumes of data (big data).

To stay current with relevant advancements, the method collects data from various sources, including scientific publications, clinical guidelines, drug inserts, and databases of clinical, blood, and genetic biomarkers. Data collection methods may include systems for continuously monitoring reputable sources such as academic journals, conference proceedings, government health agencies, and recognized mental health organizations for new publications related to mental health treatment. Application Programming Interfaces (APIs) or subscription services may be used to automatically collect this data.

Advanced text processing and natural language processing techniques are used to extract relevant information from these sources and update the parameters used in the drug selection algorithm. Natural Language Processing (NLP) techniques involve using computational methods to analyze, understand, and generate human language data. These techniques enable computers to interact with and process text or speech data, including tasks such as text classification, named entity recognition, machine translation, and text summarization. Analyzed text can include treatment modalities, therapeutic techniques, medication efficacy, side effects, patient populations, and outcome measures. NLP techniques can also be used to gather the parameters of the biomarkers.

The drug selection algorithm dynamically adapts to incorporate new evidence and medical knowledge or to to adapt to technological evolution, leveraging new tools and techniques to improve its performance. This may involve adjustments to the weights assigned to different clinical attributes, the inclusion of new drugs in the database, or the modification of decision rules based on the latest clinical recommendations. This could involve selecting suitable Artificial Intelligence (AI) models for regression tasks, considering the nature of the problem and the available data. Commonly used models for this task include Linear Regression, Decision Trees, Random Forests, Gradient Boosting Machines, Neural Networks, and Support Vector Regression. Training each selected AI model on the training dataset using drug scores calculated using the MCDA algorithm as the target variable.

Furthermore, this could involve training each selected AI model on the training dataset using the drug scores calculated using the MCDA algorithm as the target variable. The validation of proposed model improvements is goes through rigorous evaluation, including evaluating trained models on the testing dataset using appropriate evaluation metrics for regression tasks. Common metrics include Mean Squared Error, Mean Absolute Error, and R-squared. The performance of different models is compared to identify the best-performing one. Once the best-performing AI model is selected, its predictions are validated against new patient data not used during training. Model performance is continuously monitored, and the model is periodically updated using additional patient data and drug scores calculated using MCDA.

### References

ACOG Committee on Practice Bulletins--Obstetrics (2008). ACOG Practice Bulletin: Clinical management guidelines for obstetrician-gynecologists number 92, April 2008 (replaces practice bulletin number 87, November 2007). Use of psychiatric medications during pregnancy and lactation. Obstetrics and gynecology, 111(4), 1001-1020. https://doi.org/10.1097/AOG.0b013e31816fd910
Angst, J., Adolfsson, R., Benazzi, F., Gamma, A., Hantouche, E., Meyer, T., ... Scott, J. (2005). The HCL-32: Towards a self-assessment tool for hypomanic symptoms in outpatients. Journal of Affective Disorders, 88(2), 217-233. doi:10.1016/j.jad.2005.05.011.
Baldwin, D. S., Anderson, I. M., Nutt, D. J., Allgulander, C., Bandelow, B., den Boer, J. A., Christmas, D. M., Davies, S., Fineberg, N., Lidbetter, N., Malizia, A., McCrone, P., Nabarro, D., O'Neill, C., Scott, J., van der Wee, N., & Wittchen, H. U. (2014). Evidence-based pharmacological treatment of anxiety disorders, posttraumatic stress disorder and obsessive-compulsive disorder: a revision of the 2005 guidelines from the British Association for Psychopharmacology. Journal of psychopharmacology (Oxford, England), 28(5), 403-439. https://doi.org/10.1177/0269881114525674
Bandelow, B., Allgulander, C., Baldwin, D. S., Costa, D. L. D. C., Denys, D., Dilbaz, N., Domschke, K., Eriksson, E., Fineberg, N. A., Hättenschwiler, J., Hollander, E., Kaiya, H., Karavaeva, T., Kasper, S., Katzman, M., Kim, Y. K., Inoue, T., Lim, L., Masdrakis, V., Menchón, J. M., ... Zohar, J. (2023). World Federation of Societies of Biological Psychiatry (WFSBP) guidelines for treatment of anxiety, obsessive-compulsive and posttraumatic stress disorders - Version 3. Part I: Anxiety disorders. The world journal of biological psychiatry : the official journal of the World Federation of Societies of Biological Psychiatry, 24(2), 79-117. https://doi.org/10.1080/15622975.2022.2086295
Bishop, J. R., Schneiderhan, M. E., Butler, T., Carpentier, R. M., Heins, K. R., & Formea, C. M. (2023). Pharmacogenomics to support mental health medication therapy management: Clinical practice considerations and a conceptual framework to enhance patient care. JACCP: JOURNAL OF THE AMERICAN COLLEGE OF CLINICAL PHARMACY, 7(2), 160-170. doi:10.1002/jac5.1892
Bobbitt, S., Kawamura, A., Saunders, N., Monga, S., Penner, M., & Andrews, D. (02 2023). Anxiety in children and youth: Part 2-The management of anxiety disorders. Paediatrics & Child Health, 28(1), 60-66. doi:10.1093/pch/pxac104
Bousman, C. A., Bengesser, S. A., Aitchison, K. J., Amare, A. T., Aschauer, H., Baune, B. T., ... Müller, D. J. (2020). Review and Consensus on Pharmacogenomic Testing in Psychiatry. Pharmacopsychiatry, 54(01), 5-17. doi:10.1055/a-1288-1061.
Bråten, L. S., Haslemo, T., Jukic, M. M., Ingelman-Sundberg, M., Molden, E., & Kringen, M. K. (2019). Impact of CYP2C19 genotype on sertraline exposure in 1200 Scandinavian patients. Neuropsychopharmacology, 45(3), 570-576. doi:10.1038/s41386-019-0554-x
Brown, J. T., Bishop, J. R., Sangkuhl, K., Nurmi, E. L., Mueller, D. J., Dinh, J. C., ... Leeder, J. S. (2019). Clinical Pharmacogenetics Implementation Consortium Guideline for Cytochrome P450 (CYP)2D6 Genotype and Atomoxetine Therapy. Clinical Pharmacology & Therapeutics, 106(1), 94-102. doi:10.1002/cpt.1409
Carvalho Henriques, B., Yang, E. H., Lapetina, D., Carr, M. S., Yavorskyy, V., Hague, J., & Aitchison, K. J. (2020). How Can Drug Metabolism and Transporter Genetics Inform Psychotropic Prescribing? Frontiers in Genetics, 11. doi: 1 0.3389/fgene.2020.491895
Caudle, K. E., Dunnenberger, H. M., Freimuth, R. R., Peterson, J. F., Burlison, J. D., Whirl-Carrillo, M., ... Hoffman, J. M. (2017). Standardizing terms for clinical pharmacogenetic test results: consensus terms from the Clinical Pharmacogenetics Implementation Consortium (CPIC). Genetics in Medicine, 19(2), 215-223. doi:10.1038/gim.2016.87.
Chan, A. Y. L., Ma, T. T., Lau, W. C. Y., Ip, P., Coghill, D., Gao, L., Jani, Y. H., Hsia, Y., Wei, L., Taxis, K., Simonoff, E., Taylor, D., Lum, T. Y., Man, K. K. C., & Wong, I. C. K. (2023). Attention-deficit/hyperactivity disorder medication consumption in 64 countries and regions from 2015 to 2019: a longitudinal study. EClinicalMedicine, 58, 101780. https://doi.org/10.1016/j.eclinm.2022.101780
CIMA. Online Information Center for Medicines of the Spanish Agency of Medicines and Medical Devices (AEMPS) - CIMA. https://cima.aemps.es/cima/publico/home.html.
CPIC. (Accessed February 12, 2024). Guidelines. Clinical Pharmacogenetics Implementation Consortium (CPIC). Retrieved from https://cpicpgx.org/guidelines/
Cortese, S., Adamo, N., Del Giovane, C., Mohr-Jensen, C., Hayes, A. J., Carucci, S., Atkinson, L. Z., Tessari, L., Banaschewski, T., Coghill, D., Hollis, C., Simonoff, E., Zuddas, A., Barbui, C., Purgato, M., Steinhausen, H. C., Shokraneh, F., Xia, J., & Cipriani, A. (2018). Comparative efficacy and tolerability of medications for attention-deficit hyperactivity disorder in children, adolescents, and adults: a systematic review and network meta-analysis. The lancet. Psychiatry, 5(9), 727-738. https://doi.org/10.1016/S2215-0366(18)30269-4
Cox, J. H., Seri, S., & Cavanna, A. E. (2014). Clinical Guidelines on Long-Term Pharmacotherapy for Bipolar Disorder in Children and Adolescents. Journal of clinical medicine, 3(1), 135-143. https://doi.org/10.3390/jcm3010135
Dent, R., Blackmore, A., Peterson, J., Habib, R., Kay, G. P., Gervais, A., Taylor, V., & Wells, G. (2012). Changes in body weight and psychotropic drugs: a systematic synthesis of the literature. PloS one, 7(6), e36889. https://doi.org/10.1371/journal.pone.0036889
Department for Communities and Local Government. London (2009). Multicriteria Analysis: A Manual (p. 46). p. 46.
DPWG (Updated November 23, 2023. Accessed February 12 2024). Farmacogenetica. Dutch Pharmacogenetics Working Group (DPWG). Retrieved from https://www.knmp.nl/dossiers/farmacogenetica
Edwards, W., & Barron, F. H. (1994). SMARTS and SMARTER: improved simple methods for multiattribute utility measurement. pp.306-325. Organizational Behavior and Human Decision Processes.
EMC. Electronic medicines compendium. https://www.medicines.org.uk/emc
Eugene A. R. (2020). Association of sleep among 30 antidepressants: a population-wide adverse drug reaction study, 2004-2019. PeerJ, 8, e8748. https://doi.org/10.7717/peerj.8748
Fagan, H. A., & Baldwin, D. S. (2023). Pharmacological Treatment of Generalised Anxiety Disorder: Current Practice and Future Directions. Expert review of neurotherapeutics, 23(6), 535-548. https://doi.org/10.1080/14737175.2023.2211767
Faries, D. E., Yalcin, I., Harder, D., & Heiligenstein, J. H. (2001). Validation of the ADHD Rating Scale as a clinician administered and scored instrument. Journal of Attention Disorders, 5(2), 107-115. doi:10.1177/108705470100500204.
Finsterer, J., & Segall, L. (2009). Drugs interfering with mitochondrial disorders. Drug and Chemical Toxicology, 33(2), 138-151. doi:10.3109/01480540903207076.
Flockhart DA, Thacker, D., McDonald, C., Desta, Z. The Flockhart Cytochrome P450 Drug-Drug Interaction Table. Division of Clinical Pharmacology, Indiana University School of Medicine (Updated 2021). https://drug-interactions.medicine.iu.edu/.
Galletly, C., Castle, D., Dark, F., Humberstone, V., Jablensky, A., Killackey, E., Kulkarni, J., McGorry, P., Nielssen, O., & Tran, N. (2016). Royal Australian and New Zealand College of Psychiatrists clinical practice guidelines for the management of schizophrenia and related disorders. The Australian and New Zealand journal of psychiatry, 50(5), 410-472. https://doi.org/10.1177/0004867416641195
Glauser T, Wenstrup R, et al, inventors, Cincinnati Children's Hospital Medical Center, assignee. Optimization and individualization of medication selection and dosage. Spanish patent ES2529211T3. November 28, 2006.
Goldberg JF, Ernst CL. Managing the Side Effects of Psychotropic Medications. 2nd ed. American Psychiatric Association Publishing; 2019.
Gong, L., Whirl-Carrillo, M., & Klein, T. E. (2021). PharmGKB, an Integrated Resource of Pharmacogenomic Knowledge. Current Protocols, 1(8). doi:10.1 002/cpz1.226
Hale T.W. (2020). Hale's Medications & Mothers' MilkTM 2021: A Manual of Lactational Pharmacology. 19th ed. Springer Publishing Company.
Hamilton, M. (1959). THE ASSESSMENT OF ANXIETY STATES BY RATING. British Journal of Medical Psychology, 32(1), 50-55. doi:10.1111/j.2044-8341.1959.tb00467.x
Hamilton, M. (1960). A RATING SCALE FOR DEPRESSION. Journal of Neurology, Neurosurgery & Psychiatry, 23(1), 56-62. doi:10.1136/jnnp.23.1.56.
Hansen, P., & Devlin, N. (2019, April). Multi-Criteria Decision Analysis (MCDA) in Healthcare Decision-Making. Oxford Research Encyclopedia of Economics and Finance. doi:10.1093/acrefore/9780190625979.013.98.
Hasan, A., Falkai, P., Wobrock, T., Lieberman, J., Glenthøj, B., Gattaz, W. F., Thibaut, F., Möller, H. J., & WFSBP Task Force on Treatment Guidelines for Schizophrenia (2015). World Federation of Societies of Biological Psychiatry (WFSBP) Guidelines for Biological Treatment of Schizophrenia. Part 3: Update 2015 Management of special circumstances: Depression, Suicidality, substance use disorders and pregnancy and lactation. The world journal of biological psychiatry : the official journal of the World Federation of Societies of Biological Psychiatry, 16(3), 142-170. https://doi.org/10.3109/15622975.2015.1009163
Hasan, A., Falkai, P., Wobrock, T., Lieberman, J., Glenthøj, B., Gattaz, W. F., ... Möller, H.-J. (2017). World Federation of Societies of Biological Psychiatry (WFSBP) guidelines for biological treatment of schizophrenia - a short version for primary care. International Journal of Psychiatry in Clinical Practice, 21(2), 82-90. doi:10.1080/13651501.2017.1291839.
Health, T. L. G. (2020, November). Mental health matters. The Lancet Global Health, Vol. 8, p. e1352. doi:10.1016/s2214-109x(20)30432-0.
Hicks, J. K., Bishop, J. R., Sangkuhl, K., Müller, D. J., Ji, Y., Leckband, S. G., ... Gaedigk, A. (2015). Clinical Pharmacogenetics Implementation Consortium (CPIC) Guideline for CYP2D6 and CYP2C19 Genotypes and Dosing of Selective Serotonin Reuptake Inhibitors. Clinical Pharmacology & Therapeutics, 98(2), 127-134. doi:10.1002/cpt.147
Hicks, J. K., Sangkuhl, K., Swen, J. J., Ellingrod, V. L., Müller, D. J., Shimoda, K., ... Stingl, J. C. (2017). Clinical pharmacogenetics implementation consortium guideline (CPIC) for CYP2D6 and CYP2C19 genotypes and dosing of tricyclic antidepressants: 2016 update. Clinical Pharmacology & Therapeutics, 102(1), 37-44. doi:10.1002/cpt.597
Hirschfeld, R. M. A., Williams, J. B. W., Spitzer, R. L., Calabrese, J. R., Flynn, L., Keck, P. E., ... Zajecka, J. (2000). Development and Validation of a Screening Instrument for Bipolar Spectrum Disorder: The Mood Disorder Questionnaire. American Journal of Psychiatry, 157(11), 1873-1875. doi: 1 0.1176/appi.ajp.157.11.1873.
Howes, O. D., Thase, M. E., & Pillinger, T. (2021). Treatment resistance in psychiatry: state of the art and new directions. Molecular Psychiatry, 27(1), 58-72. doi:10.1038/s41380-021-01200-3.
ISPG. Genetic testing statement: genetic testing and psychiatric disorders: a statement from the International Society of Psychiatric Genetics (2019). Retrieved from http://ispg.net/genetic-testing-statement/
Jukić, M. M., Haslemo, T., Molden, E., & Ingelman-Sundberg, M. (2018). Impact of CYP2C19 Genotype on Escitalopram Exposure and Therapeutic Failure: A Retrospective Study Based on 2,087 Patients. American Journal of Psychiatry, 175(5), 463-470. doi:10.1176/appi.ajp.2017.17050550
Katzman, M. A., Bleau, P., Blier, P., Chokka, P., Kjernisted, K., Van Ameringen, M., Canadian Anxiety Guidelines Initiative Group on behalf of the Anxiety Disorders Association of Canada/Association Canadienne des troubles anxieux and McGill University, Antony, M. M., Bouchard, S., Brunet, A., Flament, M., Grigoriadis, S., Mendlowitz, S., O'Connor, K., Rabheru, K., Richter, P. M., Robichaud, M., & Walker, J. R. (2014). Canadian clinical practice guidelines for the management of anxiety, posttraumatic stress and obsessive-compulsive disorders. BMC psychiatry, 14 Suppl 1(Suppl 1), S1. https://doi.org/10.1186/1471-244X-14-S1-S1
Kay, S. R. (1990). Positive-negative symptom assessment in schizophrenia: Psychometric issues and scale comparison. Psychiatric Quarterly, 61(3), 163-178. doi:10.1007/bf01064966.
Kay, S. R., Fiszbein, A., & Opler, L. A. (1987). The Positive and Negative Syndrome Scale (PANSS) for Schizophrenia. Schizophrenia Bulletin, 13(2), 261-276. doi:10.1093/schbul/13.2.261
Kay, S. R., Opler, L. A., & Lindenmayer, J.-P. (1988). Reliability and validity of the positive and negative syndrome scale for schizophrenics. Psychiatry Research, 23(1), 99-110. doi:10.1016/0165-1781(88)90038-8
Keepers, G. A., Fochtmann, L. J., Anzia, J. M., Benjamin, S., Lyness, J. M., Mojtabai, R., Servis, M., Walaszek, A., Buckley, P., Lenzenweger, M. F., Young, A. S., Degenhardt, A., Hong, S. H., & (Systematic Review) (2020). The American Psychiatric Association Practice Guideline for the Treatment of Patients With Schizophrenia. Focus (American Psychiatric Publishing), 18(4), 493-497. https://doi.org/10.1176/appi.focus.18402
Knox, C., Wilson, M., Klinger, C. M., Franklin, M., Oler, E., Wilson, A., Pon, A., Cox, J., Chin, N. E. L., Strawbridge, S. A., Garcia-Patino, M., Kruger, R., Sivakumaran, A., Sanford, S., Doshi, R., Khetarpal, N., Fatokun, O., Doucet, D., Zubkowski, A., Rayat, D. Y., ... Wishart, D. S. (2024). DrugBank 6.0: the DrugBank Knowledgebase for 2024. Nucleic acids research, 52(D1), D1265-D1275. https://doi.org/10.1093/nar/gkad976
Kobak KA. The Hamilton Depression Rating Scale (HAMD). In: Comprehensive Handbook of Psychological Assessment, Vol. 2. Personality Assessment (Pp. 87-98). ; 2004.
Kooij, S. J., Bejerot, S., Blackwell, A., Caci, H., Casas-Brugué, M., Carpentier, P. J., Edvinsson, D., Fayyad, J., Foeken, K., Fitzgerald, M., Gaillac, V., Ginsberg, Y., Henry, C., Krause, J., Lensing, M. B., Manor, I., Niederhofer, H., Nunes-Filipe, C., Ohlmeier, M. D., Oswald, P., ... Asherson, P. (2010). European consensus statement on diagnosis and treatment of adult ADHD: The European Network Adult ADHD. BMC psychiatry, 10, 67. https://doi.org/10.1186/1471-244X-10-67
Korkatti-Puoskari, N., Tiihonen, M., Caballero-Mora, M. A., Topinkova, E., Szczerbińska, K., Hartikainen, S., & on the Behalf of the EuGMS Task & Finish group on FRIDs (2023). Therapeutic dilemma's: antipsychotics use for neuropsychiatric symptoms of dementia, delirium and insomnia and risk of falling in older adults, a clinical review. European geriatric medicine, 14(4), 709-720. https://doi.org/10.1007/s41999-023-00837-3
Kroenke, K., Spitzer, R. L., & Williams, J. B. W. (2001). The PHQ-9: Validity of a brief depression severity measure. Journal of General Internal Medicine, 16(9), 606-613. doi:10.1046/j.1525-1497.2001.016009606.x.
Lee, E. S., Kronsberg, H., & Findling, R. L. (2020). Psychopharmacologic Treatment of Schizophrenia in Adolescents and Children. Child and adolescent psychiatric clinics of North America, 29(1), 183-210. https://doi.org/10.1016/j.chc.2019.08.009
Levey, A. S., Stevens, L. A., Schmid, C. H., Zhang, Y. (lucy), Castro, A. F., Feldman, H. I., ... Coresh, J. (2009). A New Equation to Estimate Glomerular Filtration Rate. Annals of Internal Medicine, 150(9), 604. doi:10.7326/0003-4819-150-9-200905050-00006.
Li, L., Sujan, A. C., Butwicka, A., Chang, Z., Cortese, S., Quinn, P., Viktorin, A., Oberg, A. S., D'Onofrio, B. M., & Larsson, H. (2020). Associations of Prescribed ADHD Medication in Pregnancy with Pregnancy-Related and Offspring Outcomes: A Systematic Review. CNS drugs, 34(7), 731-747. https://doi.org/10.1007/s40263-020-00728-2
Lynch., S. S. (Reviewed/Revised Jul 2022. Accessed October 2 2024). Overview of Response to Drugs. MSD Manual Consumer Version. Retrieved from https://www.msdmanuals.com/home/drugs/factors-affecting-response-to-drugs/overview-of-response-to-drugs.
MacQueen, G. M., Frey, B. N., Ismail, Z., Jaworska, N., Steiner, M., Lieshout, R. J., Kennedy, S. H., Lam, R. W., Milev, R. V., Parikh, S. V., Ravindran, A. V., & CANMAT Depression Work Group (2016). Canadian Network for Mood and Anxiety Treatments (CANMAT) 2016 Clinical Guidelines for the Management of Adults with Major Depressive Disorder: Section 6. Special Populations: Youth, Women, and the Elderly. Canadian journal of psychiatry. Revue canadienne de psychiatrie, 61(9), 588-603. https://doi.org/10.1177/0706743716659276
Maier, W., Buller, R., Philipp, M., & Heuser, I. (1988). The Hamilton Anxiety Scale: reliability, validity and sensitivity to change in anxiety and depressive disorders. Journal of Affective Disorders, 14(1), 61-68. doi:10.1016/0165-0327(88)90072-9.
Malhi, G. S., Bell, E., Bassett, D., Boyce, P., Bryant, R., Hazell, P., Hopwood, M., Lyndon, B., Mulder, R., Porter, R., Singh, A. B., & Murray, G. (2021). The 2020 Royal Australian and New Zealand College of Psychiatrists clinical practice guidelines for mood disorders. The Australian and New Zealand journal of psychiatry, 55(1), 7-117. https://doi.org/10.1177/0004867420979353
Manning D.R., & Blumenthal D.K. Pharmacodynamics: molecular mechanisms of drug action. Brunton L.L., & Knollmann B.C.(Eds.), 2023 Goodman & Gilman's: The Pharmacological Basis of Therapeutics, 14th Edition. McGraw-Hill Education. https://accessmedicine.mhmedical.com/content.aspx?bookid=3191&sectionid=2697154 60.
McMahon F, Laje G, et al, inventors, US Department of Health and Human Services University of Texas System, assignee. Methods to predict the outcome of treatment with antidepressant medication. United States patent US7795033B2. March 19, 2008.
Mendlewicz, J. (2008). Towards achieving remission in the treatment of depression. Dialogues in Clinical Neuroscience, 10(4), 371-375. doi:10.31887/dcns.2008.10.4/jmendlewicz.
Mrazek A, O'Kane D, et al, inventors, Mayo Foundation for Medical Education and Research, assignee. Methods for selecting medications. United States patent US20200048712A1. May 15, 2019.
Nasser, A., Kosheleff, A. R., Hull, J. T., Liranso, T., Qin, P., Busse, G. D., ... Rubin, J. (2021). Translating Attention-Deficit/Hyperactivity Disorder Rating Scale-5 and Weiss Functional Impairment Rating Scale-Parent Effectiveness Scores into Clinical Global Impressions Clinical Significance Levels in Four Randomized Clinical Trials of SPN-812 (Viloxazine Extended-Release) in Children and Adolescents with Attention-Deficit/Hyperactivity Disorder. Journal of Child and Adolescent Psychopharmacology, 31(3), 214-226. doi: 10.1 089/cap.2020.0148
Ng, I. K. S., Chua, J. W., Lui, Y. S., Tan, L. F., & Teo, D. B. S. (2023). Approach to acute psychosis in older adults. Singapore medical journal, 64(6), 391-396. https://doi.org/10.4103/singaporemedj.SMJ-2022-150
National Institute for Health and Care Excellence (NICE) (2019). Attention deficit hyperactivity disorder: diagnosis and management. London.
Nofziger, C., Turner, A. J., Sangkuhl, K., Whirl-Carrillo, M., Agúndez, J. A. G., Black, J. L., ... Gaedigk, A. (2019). PharmVar GeneFocus: CYP2D6. Clinical Pharmacology & Therapeutics, 107(1), 154-170. doi:10.1002/cpt.1643.
OECD. (2023). Health at a Glance 2023: OECD Indicators. doi:10.1787/7a7afb35-en.
Organization, W. H. (2022). World mental health report: transforming mental health for all. World Health Organization.
Owen, R. P., Sangkuhl, K., Klein, T. E., & Altman, R. B. (2009). Cytochrome P450 2D6. Pharmacogenetics and Genomics, 19(7), 559-562. doi:10.1097/fpc.0b013e32832e0e97.
Pernia, S., & DeMaagd, G. (2016). The New Pregnancy and Lactation Labeling Rule. P & T : a peer-reviewed journal for formulary management, 41(11), 713-715.
PharmVar (n.d.) Pharmacogene Variation Consortium. Genes. https://www.pharmvar.org/genes
Pratt, V. M., Cavallari, L. H., Fulmer, M. L., Gaedigk, A., Hachad, H., Ji, Y., ... Weck, K. E. (2023). CYP3A4 and CYP3A5 Genotyping Recommendations. The Journal of Molecular Diagnostics, 25(9), 619-629. doi:10.1016/j.jmoldx.2023.06.008.
Radosavljevic, M., Svob Strac, D., Jancic, J., & Samardzic, J. (2023). The Role of Pharmacogenetics in Personalizing the Antidepressant and Anxiolytic Therapy. Genes, 14(5), 1095. doi:10.3390/genes14051095
Royal College of Psychiatrists (2023) College Report CR235 ADHD in adults: Good practice guidance.
Schatzberg AF, DeBattista C. Schatzberg's Manual of Psychopharmacology. American Psychiatric Association Publishing; 2019. doi:10.1176/appi.books.9781615372997.
Scott, S. A., Sangkuhl, K., Shuldiner, A. R., Hulot, J.-S., Thorn, C. F., Altman, R. B., & Klein, T. E. (2012). PharmGKB summary: very important pharmacogene information for cytochrome P450, family 2, subfamily C, polypeptide 19. Pharmacogenetics and Genomics, 22(2), 159-165. doi:10.1097/fpc.0b013e32834d4962.
Seo, J. S., Bahk, W. M., Woo, Y. S., Park, Y. M., Kim, W., Jeong, J. H., Shim, S. H., Lee, J. G., Jang, S. H., Yang, C. M., Wang, S. M., Jung, M. H., Sung, H. M., Choo, I. H., Yoon, B. H., Lee, S. Y., Jon, D. I., & Min, K. J. (2021). Korean Medication Algorithm for Depressive Disorder 2021, Fourth Revision: An Executive Summary. Clinical psychopharmacology and neuroscience : the official scientific journal of the Korean College of Neuropsychopharmacology, 19(4), 751-772. https://doi.org/10.9758/cpn.2021.19.4.751
Strawn, J. R., Geracioti, L., Rajdev, N., Clemenza, K., & Levine, A. (2018). Pharmacotherapy for generalized anxiety disorder in adult and pediatric patients: an evidence-based treatment review. Expert opinion on pharmacotherapy, 19(10), 1057-1070. https://doi.org/10.1080/14656566.2018.1491966
Swen, J. J., Nijenhuis, M., de Boer, A., Grandia, L., Maitland-van der Zee, A. H., Mulder, H., ... Guchelaar, H.-J. (2011). Pharmacogenetics: From Bench to Byte- An Update of Guidelines. Clinical Pharmacology & Therapeutics, 89(5), 662-673. doi:10.1038/clpt.2011.34
Tansey, K. E., Guipponi, M., Hu, X., Domenici, E., Lewis, G., Malafosse, A., Uher, R. (2013). Contribution of Common Genetic Variants to Antidepressant Response. Biological Psychiatry, 73(7), 679-682. doi:10.1016/j.biopsych.2012.10.030.
Taylor, S., Zvolensky, M. J., Cox, B. J., Deacon, B., Heimberg, R. G., Ledley, D. R., ... Cardenas, S. J. (2007). Robust dimensions of anxiety sensitivity: Development and initial validation of the Anxiety Sensitivity Index-3. Psychological Assessment, 19(2), 176-188. doi:10.1037/1040-3590.19.2.176.
Therapeutic Goods Administration (TGA). Australian Government. Department of Health and Aged Care. Prescribing medicines in pregnancy database. The Australian categorization system for prescribing medicines in pregnancy. https://webarchive.nla.gov.au/awa/20220815154145/https://www.tga.gov.au/prescribing -medicines-pregnancy-database.
Thorn, C. F., Lamba, J. K., Lamba, V., Klein, T. E., & Altman, R. B. (2010). PharmGKB summary: very important pharmacogene information for CYP2B6. Pharmacogenetics and Genomics, 20(8), 520-523. doi:10.1097/fpc.0b013e32833947c2.
Thorn, C. F., Aklillu, E., Klein, T. E., & Altman, R. B. (2012). PharmGKB summary: very important pharmacogene information for CYP1A2. Pharmacogenetics and Genomics, 22(1), 73-77. doi:10.1097/fpc.0b013e32834c6efd.
Van Booven, D., Marsh, S., McLeod, H., Carrillo, M. W., Sangkuhl, K., Klein, T. E., & Altman, R. B. (2010). Cytochrome P450 2C9-CYP2C9. Pharmacogenetics and Genomics, 20(4), 277-281. doi:10.1097/fpc.0b013e3283349e84.
Van Westrhenen, R., & Ingelman-Sundberg, M. (2021). Editorial: From Trial and Error to Individualized Pharmacogenomics-Based Pharmacotherapy in Psychiatry. Frontiers in Pharmacology, 12. doi:10.3389/fphar.2021.725565.
Walter, H. J., Abright, A. R., Bukstein, O. G., Diamond, J., Keable, H., Ripperger-Suhler, J., & Rockhill, C. (2023). Clinical Practice Guideline for the Assessment and Treatment of Children and Adolescents With Major and Persistent Depressive Disorders. Journal of the American Academy of Child and Adolescent Psychiatry, 62(5), 479-502. https://doi.org/10.1016/j.jaac.2022.10.001
Whirl-Carrillo, M., McDonagh, E. M., Hebert, J. M., Gong, L., Sangkuhl, K., Thorn, C. F., Altman, R. B., & Klein, T. E. (2012). Pharmacogenomics knowledge for personalized medicine. Clinical pharmacology and therapeutics, 92(4), 414-417. https://doi.org/10.1038/clpt.2012.96
Whirl-Carrillo, M., Huddart, R., Gong, L., Sangkuhl, K., Thorn, C. F., Whaley, R., & Klein, T. E. (2021). An Evidence-Based Framework for Evaluating Pharmacogenomics Knowledge for Personalized Medicine. Clinical pharmacology and therapeutics, 110(3), 563-572. https://doi.org/10.1002/cpt.2350
Wolraich, M. L., Hagan, J. F., Jr, Allan, C., Chan, E., Davison, D., Earls, M., Evans, S. W., Flinn, S. K., Froehlich, T., Frost, J., Holbrook, J. R., Lehmann, C. U., Lessin, H. R., Okechukwu, K., Pierce, K. L., Winner, J. D., Zurhellen, W., & SUBCOMMITTEE ON CHILDREN AND ADOLESCENTS WITH ATTENTION-DEFICIT/HYPERACTIVE DISORDER (2019). Clinical Practice Guideline for the Diagnosis, Evaluation, and Treatment of Attention-Deficit/Hyperactivity Disorder in Children and Adolescents. Pediatrics, 144(4), e20192528. https://doi.org/10.1542/peds.2019-2528
Yatham, L. N., Kennedy, S. H., Parikh, S. V., Schaffer, A., Bond, D. J., Frey, B. N., Sharma, V., Goldstein, B. I., Rej, S., Beaulieu, S., Alda, M., MacQueen, G., Milev, R. V., Ravindran, A., O'Donovan, C., Mclntosh, D., Lam, R. W., Vazquez, G., Kapczinski, F., McIntyre, R. S., ... Berk, M. (2018). Canadian Network for Mood and Anxiety Treatments (CANMAT) and International Society for Bipolar Disorders (ISBD) 2018 guidelines for the management of patients with bipolar disorder. Bipolar disorders, 20(2), 97-170. https://doi.org/10.1111/bdi.12609
Young, R. C., Biggs, J. T., Ziegler, V. E., & Meyer, D. A. (1978). A Rating Scale for Mania: Reliability, Validity and Sensitivity. British Journal of Psychiatry, 133(5), 429-435. doi:10.1192/bjp.133.5.429.

## Claims

1. **METHOD FOR THE SELECTION OF DRUGS FOR THE TREATMENT OF MENTAL HEALTH PATHOLOGIES** **characterized by** comprising the following steps:
a- Three groups of biomarkers are obtained from a patient through i) a group of Biomarkers 1, ii) a group of Biomarkers 2, and iii) a group of Biomarkers 3;
where
- The Biomarkers Group 1 corresponds to the patient's clinical parameters relevant to the treatment of each pathology and its differential diagnosis;
- The Biomarkers Group 2 corresponds to the patient's blood parameters relevant to the pharmacological response;
- The Biomarkers Group 3 corresponds to the patient's genetic conditions relevant to pharmacological treatment;
b- Taking into account the values of Biomarkers Groups 1, 2, and 3; attributes are determined for a Multicriteria Decision Analysis;
c- Based on the results of the previous step, the weight of each attribute for the Multicriteria Decision Analysis is determined;
d- Considering the previous results, scores are assigned to each attribute for each drug of interest;
e- A final calculation of the score for each drug is carried out by multiplying the scores of each attribute by its weight and summing the obtained values; and
f- A list of drugs is provided, among which those with higher scores have a higher probability of response and a lower risk of adverse effects for the patient.

2. The **METHOD** according to claim 1, **characterized in that** the mental health pathologies to be treated belong to the group consisting of Major Depressive Disorder (MDD), Bipolar Disorder (BD), Attention Deficit Disorder (ADD), Psychosis (PS), Generalized Anxiety Disorder (GAD), and Social Anxiety Disorder (SAD).

3. The **METHOD** according to claim 1, **characterized in that** Biomarkers Group 1 comprises the patient's self-reported clinical parameters and the clinical parameters reported by the medical professional in charge of the patient's treatment.

4. The **METHOD** according to claim 2, **characterized in that** Biomarkers Group 1 comprises the contribution of, at least, selected clinical parameters from the group comprising: Patient Identification, Gender, Age, Pregnancy, Age Range, Underlying Pathology, Bipolar Spectrum, Body Mass Index (BMI), Sleep Disorder, Suicidal Ideation, Severity of Pathology, Weight Change, Smoking, Cognitive Effects, Previous Medications, Current Medications, Medication-induced Adverse Events, Information on whether the patient is experiencing a subtherapeutic response with current medication.

5. The **METHOD** according to claim 1, **characterized in that** Biomarkers Group 2 comprises the consideration of, at least, the following parameters: Decreased Hematocrit, Decreased Hemoglobin, Values consistent with Anemia, Values consistent with Leukopenia, Elevated Hematocrit, Values consistent with Liver Dysfunction, Values consistent with Liver Failure, Values consistent with Renal Dysfunction, Values consistent with Hypothyroidism, Values consistent with Hyperthyroidism, Values consistent with Renal Failure, Values consistent with Risk of Bleeding, Values consistent with Risk of Venous Thromboembolism, and Values consistent with Hyponatremia.

6. The **METHOD** according to claim 1, **characterized in that**, within Biomarkers Group 3, genes relevant to drug treatment in psychiatry can be classified into those with pharmacokinetic effects and those with pharmacodynamic effects.

7. The **METHOD** according to claim 6, **characterized in that** a gene panel is designed comprising genes involved in pharmacokinetics, encoding enzymes that metabolize neuropsychiatric drugs, and genes involved in pharmacodynamics, encoding molecules correlated with therapeutic response and adverse effects by their action on the target organ.

8. The **METHOD** according to claim 7, **characterized in that**, within Biomarkers Group 3, the pharmacokinetic gene panel, the pharmacodynamic gene panel, and uridine diphosphate glucuronosyltransferases are analyzed.

9. The **METHOD** according to claim 1, **characterized in that** the drugs involved are selected from the group of drugs consisting of anxiolytics, mood stabilizers, antiepileptics, antidepressants, antipsychotics, stimulants, non-stimulants, anesthetics, and adjuvant agents.

10. The **METHOD** according to claim 1, **characterized in that** the attributes considered are at least Age Range (AR), Blood Parameters (BP), Pharmacokinetics (PK), Pharmacodynamics (PD), Side Effects (SE), Pregnancy (PR), Underlying Pathology (UP), and Interaction with other drugs (DI).

11. The **METHOD** according to claim 9, **characterized in that** weights are assigned to each attribute according to the following table calculated using the simple technique of multiple attribute scoring.
| UP | PR | AR | PK | BP | PD | ES |
|---|---|---|---|---|---|---|
| | | 0,38⁽¹⁾ | 0,32 | 0,23 | 0,07 | |
| | | 0,30⁽²⁾ | 0,27 | 0,18 | 0,05 | 0,20 |
| | 0,30 | 0,20 | 0,27 | 0,18 | 0,05 | |
| 0,28 | | 0,22⁽³⁾ | 0,25 | 0,18 | 0,07 | |
| 0,25 | | 0,17⁽⁴⁾ | 0,22 | 0,15 | 0,05 | 0,16 |
| 0,20 | 0,30 | 0,11 | 0,20 | 0,14 | 0,05 | |
| | | | | | | |
|---|---|---|---|---|---|---|
| ⁽¹⁾ pediatric or geriatric , ⁽²⁾ adult, ⁽³⁾ pediatric or geriatric and ⁽⁴⁾ adult | | | | | | |

12. The **METHOD** according to claim 11, **characterized in that** scores are assigned for each attribute based on the suitability of the drug for the individual analyzed **in that** specific attribute, for example:
| | **UC** | **PR** | **AR** | **PK** |
|---|---|---|---|---|
| **Higher scores** | Drugs recommended by clinical guidelines and scientific literature for the treatment of depression in patients with the patient's underlying pathology. | Drugs recommended by clinical guidelines for the treatment of depression in pregnant women. | Drugs recommended by clinical guidelines for the treatment of depression in the age range of the individual (pediatric, adult, or geriatric). | Drugs whose main metabolism pathway is not altered. |
| **Lower scores** | Drugs not recommended by clinical guidelines and scientific literature for the treatment of depression in patients with the patient's underlying pathology. | Drugs not recommended by the guidelines **in that** specific population. | Drugs not recommended by clinical guidelines for the treatment of depression in the age range of the individual (pediatric, adult, or geriatric). | Drugs whose main metabolic pathway is altered and an alternative drug is recommended. |
| | **BP** | **PD** | **SE** | **DI** |
|---|---|---|---|---|
| **Higher scores** | Drugs without specific indication in the Electronic Medicines Compendium (EMC) for the treatment of depression in patients with altered blood parameters. | Drugs that, according to scientific literature compiled in PharmaGKB, are more effective and have lower toxicity risk given the patient's | Drugs recommended by scientific literature for the combination of muscle mass index and weight variation and secondary sleep disorder to the depressive episode of the patient. | Drugs with lower risk or severity of interaction between each other. |
| | | genetic variations. | | |
| **Lower scores** | Drugs not recommended in the Electronic Medicines Compendium (EMC) for the treatment of depression in patients with altered blood parameters. | Drugs that, according to scientific literature compiled in PharmaGKB, are less effective and have a higher risk of toxicity due to the patient's genetic variations. | Drugs not recommended by scientific literature for the combination of muscle mass index and weight variation and secondary sleep disorder to the depressive episode of the patient. | Drugs with a higher risk or severity of interaction between each other. |

13. The **METHOD** according to claim 1, **characterized in that** the parameters obtained from Biomarkers Groups 1, 2, and 3 are selected and weighted using machine learning techniques provided by Artificial Intelligence (AI), based on the data and performance of the Multicriteria Decision Analysis model used.

14. The **METHOD** according to claim 13, **characterized in that** the training of AI to achieve the required machine learning is carried out with the data obtained through the algorithm executed in Multicriteria Decision Analysis mode.
